# EUROPEAN PATENT SPECIFICATION

(11) **EP 1 238 070 B1**
(45) Date of publication and mention of the grant of the patent: **15.08.2007**
(21) Application number: 00981117.5
(22) Date of filing: 12.12.2000
(51) Int. Cl.: C12N 15/11, A61K 31/7088, A61P 35/00

(54) **THERAPEUTICALLY USEFUL SYNTHETIC OLIGONUCLEOTIDES**
SYNTHETISCHE OLIGONUKLEOTIDE DIE THERAPEUTISCH NÜTZLICH SIND
OLIGONUCLEOTIDES SYNTHETIQUES UTILES DU POINT DE VUE THERAPEUTIQUE

(30) Priority: 13.12.1999 US 170325 P; 29.08.2000 US 228925 P
(43) Date of publication of application: 11.09.2002
(73) Proprietor: Bioniche Life Sciences Inc., Belleville, Ontario K8N 5J2 (CA)
(72) Inventor: PHILLIPS, Nigel, C., Pointe-Claire, Quebec H2R 1J6 (CA); FILION, Mario, C., Laval,Québec H7E 3Y6 (CA)
(74) Representative: Dey, Michael
(86) International application number: PCT/CA2000/001467
(87) International publication number: WO 2001/044465

(56) References cited:
- WO-A-02/18593
- BATES PAULA J ET AL: "Antiproliferative activity of G-rich oligonucleotides correlates with protein binding." JOURNAL OF BIOLOGICAL CHEMISTRY, vol. 274, no. 37, 10 September 1999 (1999-09-10), pages 26369-26377, XP002168738 ISSN: 0021-9258
- KUTOH, E. ET AL.: "Genomic structure and regulation of the promoter of the rat insulin-like growth factor binding protein-2 gene." MOLECULAR ENDOCRINOLOGY, vol. 7, no. 9, 1993, pages 1205-1216, XP002324155 ISSN: 0888-8809
- FILION M C ET AL: "Mycobacterium phlei cell wall complex directly induces apoptosis in human bladder cancer cells." BRITISH JOURNAL OF CANCER, vol. 79, no. 2, January 1999 (1999-01), pages 229-235, XP001002272

## Description

### FIELD OF THE INVENTION

The present invention relates to an oligonucleotide composition for the treatment of cancer.

### BACKGROUND OF THE INVENTION

Cancer is an aberrant net accumulation of atypical cells, which can result from an excess of proliferation, an insufficiency of cell death, or a combination of the two.

Proliferation is the culmination of a cell's progression through the cell cycle resulting in the division of one cell into two cells. The 5 major phases of the cell cycle are G₀, G₁, S, G₂ and M. During the G₀ phase, cells are quiescent. Most cells in the body, at any one time, are in this stage. During the G₁ phase, cells, responding to signals to divide, produce the RNA and the proteins necessary for DNA synthesis. During the S-phase (SE, early S-phase; SM, middle S-phase; and SL, late S-phase) the cells replicate their DNA. During the G₂ phase, proteins are elaborated in preparation for cell division. During the mitotic (M) phase, the cell divides into two daughter cells. Alterations in cell cycle progression occur in all cancers and may result from over-expression of genes, mutation of regulatory genes, or abrogation of DNA damage checkpoints (Hochhauser D. Anti-Cancer Chemotherapeutic Agents 8:903, 1997).

Unlike cancer cells, most normal cells cannot proliferate indefinitely due to a process termed cellular senescence. Cellular senescence is a programmed cell death response leading to growth arrest of cells (Dimri et al. Proc. Natl. Acad. Sci. USA 92:20, 1995). DNA damage, exposure of colon, breast and ovarian cancer cells to toposiomerase inhibitors and exposure of nasopharyngeal cancer cells to cisplatin are reported to prevent proliferation of these cells by induction of senescence (Wang et al. Cancer Res. 58:5019, 1998; Poele et al. Br. J. Cancer 80:9, 1999).

Synthetic oligonucleotides are polyanionic sequences that are internalized in cells (Vlassov et al. Biochim. Biophys. Acta 1197:95, 1994). Synthetic oligonucleotides are reported that bind selectively to nucleic acids (Wagner, R. Nature: 372:333, 1994), to specific cellular proteins (Bates et al. J. Biol. Chem. 274:26369, 1999) and to specific nuclear proteins Scaggiante et al. Eur. J. Biochem. 252:207, 1998) to inhibit proliferation of cancer cells.

Synthetic 27 base sequences containing guanine (G) and variable amounts of thymine (T) (oligonucleotide GTn), wherein n is ≥ 1 or ≤ 7 and wherein the number of bases is ≥20 (Scaggiante et al. Eur. J. Biochem. 252:207, 1998), are reported to inhibit growth of cancer cell lines by sequence specific binding to a 45 kDa nuclear protein, whereas GTn, wherein the number of bases is ≤20, are reported to be inactive against cancer cell lines (Morassutti et al. Nucleosides and Nucleotides 18:1711, 1999). Two synthetic GT-rich oligonucleotides of 15 and 29 bases with 3' aminoalkyl modifications are reported to form G-quartets that bind to nucleolin and to inhibit proliferation of cancer cell lines (Bates et al. J. Biol. Chem. 274:26369, 1999). The synthetic 6 base TTAGGG-phosphorothioate, having a sequence identical to that of the mammalian telomere repeat sequence, is reported to inhibit proliferation of Burkitt's lymphoma cells *in vitro* and in vivo (Mata et al. Toxicol. Applied Pharmacol. 144:189, 1997). However, the synthetic 6 base TTAGGG-phosphodiester is reported to have no anti-telomerase activity (US Patent No:5,643,890).

Cell death is effected by immune-mediators that promote apoptosis, and by apoptosis inducers that directly initiate pathways leading to cell death (Muzio et al. Cell 85:817, 1996; Levine, A. Cell 88:323, 1997). Apoptosis is an active cellular death process characterized by distinctive morphological changes that include condensation of nuclear chromatin, cell shrinkage, nuclear disintegration, plasma membrane blebbing, and the formation of membrane-bound apoptotic bodies (Wyllie et al. Int. Rev. Cytol. 68:251, 1980). A molecular hallmark of apoptosis is degradation of the cell's nuclear DNA into oligonucleosomal-length fragments as the result of activation of endogenous endonucleases (Wyllie A. Nature 284:555, 1980).

Caspases (cysteine-aspartyl-specific proteases) have been implicated as key enzymes in the execution of the late stage of apoptosis. The caspase family consists of at least fourteen related cysteine aspartyl proteases. All the caspases contain a conserved QACXG (where X is R, Q or G) pentapeptide active-site motif (Cohen G. Biochim. Biophys. Acta 1366:139, 1997). A number of caspases are synthesized as inactive proenzymes that are activated following cleavage at caspase specific cleavage sites (Cohen G. Biochim. Biophys. Acta 1366:139, 1997) or as inactive enzymes that require association with regulatory molecules for activation (Stennicke et al. J. Biol. Chem. 274:8359, 1999).

In addition to their role in apoptosis, caspases are involved in activation and proliferation of B and T lymphocytes, in cytokine maturation during inflammation, in differentiation of progenitor cells during erythropoiesis and in development of lens fiber (Fadeel et al. Leukemia 14:1514, 2000). With respect to B and T lymphocytes, caspase 3 is processed during activation of B lymphocytes and of CD4 (+), CD8 (+), CD45RA(+) and CD45RO (+) subsets of T lymphocytes (Alam et al. J. Exp. Med. 190:1879, 1999). Moreover, stimulation of T lymphocytes by mitogens and by interleukin-2 is associated with activation of the caspase pathway and with cleavage of PARP (Wilheim et al. Eur. J. Immunol. 28:891, 1998). With respect to cytokines, caspase 3 activity is necessary for the release of IL-2 by activated T lymphocytes (Posmantur et al. Exp. Cell Res. 244:302, 1998) and for the processing and maturation of the pro-inflammatory cytokine interleukin-16 (Zhang et al. J. Biol. Chem. 273:1144, 1998). With respect to erythropoiesis, caspase activation is involved in erythropoiesis regulation and has been shown to modulate GATA-1, a nuclear regulatory protein crucial for the maturation of erythroid precursors (De Maria, et al. Nature 401:489, 1999).

Cytolysis is the complete or partial destruction of a cell and is mediated by the immune system. Activated macrophages and monocytes produce bioactive molecules that include, but are not limited to cytokines. Cytokines, include, but are not limited to, interleukin (IL)-1, IL-1 beta, IL-6, IL-10, IL-12, and TNF-alpha.

IL-1beta reduces bone marrow cell sensitivity to cytoreductive drugs, to radiation and to *in vitro* tumor cell purging with drugs in autologous bone marrow transplantation (Dalmau et al. Bone Marrow Transplant. 12:551, 1993).

IL-6 induces B cell differentiation, stimulates IgG secretion (Taga et al. J. Exp. Med. 166:967, 1987), induces cytotoxic T cell differentiation (Lee et al. Vaccine 17:490, 1999), promotes megakaryocyte maturation (Ishibashi et al. Proc. Natl. Acad. Sci. USA 86:8953, 1989) and functions both as an anti-proliferative factor (Mori et al. Biochem. Biophys. Res. Comm. 257:609, 1999; Alexandroff et al. Biochem. Soc. Trans. 25:270, 1997; Takizawa et al. Cancer Res. 53:18, 1993: Novick et al. Cytokine 4:6, 1992) and as a pro-proliferative factor (Okamoto et al. Cancer Res. 57:141, 1997; Okamoto et al. Int. J. Cancer 72:149, 1997; Chiu et al. Clin. Cancer Res. 2:215, 1996; Lu et al. Clin. Cancer Res. 2:1417, 1996) for cancer cells.

IL-10 enhances the effectiveness of vaccines in murine tumor models (Kauffman et al. J. Immunother. 22: 489, 1999) and up-regulates anti-cancer autoreactive T cell responses (Alleva et al. Immunobiol. 192:155,1995).

IL-12, alone and in combination with other cytokines, promotes the maturation of leukocytes and induces the secretion of interferon-gamma. IL-12 is reported to have anti-cancer activity (Stine et al. Annals NY Academy of Science 795:420, 1996; Chen et al. Journal of Immunol. 159:351, 19977 including, but not limited to, activation of specific cytolytic T-lymphocytes, activation of natural killer (NK) cells and induction of the anti-angiogenic proteins IP-10 and MiG.

TNF-alpha causes necrosis of solid tumors (Porter et al. Trends in Biotech. 9:158, 1991), sensitizes cancer cells to gamma irradiation-induced apoptosis (Kimura et al. Cancer Res. 59:1606, 1999) and protects bone marrow precursor cells from the effects of antineoplastic agents (Dalmau et al. Bone Marrow Transplant. 12:551, 1993).

However, most prior art anti-cancer therapies, whether directed to induction of cell cycle arrest, inhibition of proliferation, induction of apoptosis or stimulation of the immune system, have proven to be less than adequate for clinical applications. Many of these therapies are inefficient or toxic, have significant adverse side effects, result in development of drug resistance or immunosensitization, and are debilitating for the recipient.

Therefore, there is a continuing need for novel compositions and methods that induce cell cycle arrest in cancer cells, inhibit proliferation of cancer cells, activate caspases in cancer cells, induce apoptosis in cancer cells and stimulate cytokine production by immune system cells.

### SUMMARY OF THE INVENTION

The present invention fulfills this need by providing a composition and method comprising a 3'-OH, 5'-OH synthetic oligonucleotide of SEQ ID NO:45: gggagg and wherein the sequence induces a response selected from the group consisting of induction of cell cycle arrest, inhibition of proliferation, activation of caspases and induction of apoptosis in cancer cells and production of cytokines by immune system cells. A composition comprising the sequence SEQ ID NO:45 and a pharmaceutically acceptable carrier is administered to an animal, including a human, having cancer in an amount effective to treat the cancer in the animal. The unexpected and surprising ability of the sequence to induce cell cycle arrest, inhibit proliferation, activate caspases and induce apoptosis and in cancer cells and to stimulate cyotkine production by immune system cells addresses a long felt unfulfilled need in the medical arts and provides an important benefit for animals, including humans.

Accordingly, it is an object of the present invention is to provide a composition and method effective to treat a disease in an animal, including a human.

Another object of the present invention is to provide a composition and method effective to treat a cancer.

Another object of the present invention is to provide a composition and method that induces senescence in cells.

Another object of the present invention is to provide a composition and method that induces cell cycle arrest in cells.

Another object of the present invention is to provide a composition and method that induces cell cycle arrest in cancer cells.

Another object of the present invention is to provide a composition and method that inhibits proliferation of cells.

Another object of the present invention is to provide a composition and method that inhibits proliferation of cancer cells.

Another object of the present invention is to provide a composition and method that induces apoptosis in cells.

Another object of the present invention is to provide a composition and method that induces apoptosis in cancer cells.

Another object of the present invention is to provide a composition and method that induces apoptosis in cancer cells independent of Fas.

Another object of the present invention is to provide a composition and method that induces apoptosis in cancer cells independent of TNFR1.

Another object of the present invention is to provide a composition and method that induces apoptosis in cancer cells independent of p53/p21.

Another object of the present invention is to provide a composition and method that induces apoptosis in cancer cells independent of p21/waf-1/CIP.

Another object of the present invention is to provide a composition and method that induces apoptosis in cancer cells independent of p15^{ink4B}.

Another object of the present invention is to provide a composition and method that induces apoptosis in cancer cells independent of p16^{ink4}.

Another object of the present invention is to provide a composition and method that induces apoptosis in cancer cells independent of caspase 3.

Another object of the present invention is to provide a composition and method that induces apoptosis in cancer cells independent of TGF-beta 1 receptor.

Another object of the present invention is to provide a composition and method that induces apoptosis in cancer cells independent of hormone dependence.

Another object of the present invention is to provide a composition and method that induces apoptosis in cancer cells independent of drug resistance.

Another object of the present invention is to provide a composition and method that activates caspases in cells.

Another object of the present invention is to provide a composition and method that activates caspases in cancer cells.

Another object of the present invention is to provide a composition and method to treat an autoimmune disease.

Another object of the present invention is to provide a composition and method to treat a lymphoproliferative disease.

Another object of the present invention is to provide a composition and method to treat an infection.

Another object of the present invention is to provide a composition and method to treat an inflammation.

Another object of the present invention is to provide a composition and method to modulate T- or B-cell activation.

Another object of the present invention is to provide a composition and method to modulate progenitor cell maturation.

Another object of the present invention is to provide a composition and method to modulate erythropoiesis.

Another object of the present invention is to provide a composition and method to modulate transcription factors in cells.

Another object of the present invention is to provide a composition and method that potentiates the effect of other therapeutic agents on cells.

Another object of the present invention is to provide a composition and method that potentiates the effect of chemotherapeutic agents on cancer cells.

Another object of the present invention is to provide a composition and method that potentiates the anti-neoplastic effect of radiation.

Another object of the present invention is to provide a composition and method that stimulates cytokine production by cells of the immune system.

Another object of the present invention is to provide a composition and method that stimulates IL-1beta production by cells of the immune system.

Another object of the present invention is to provide a composition and method that stimulates IL-6 production by cells of the immune system.

Another object of the present invention is to provide a composition and method that stimulates IL-10 production by cells of the immune system.

Another object of the present invention is to provide a composition and method that stimulates IL-12 production by cells of the immune system.

Another object of the present invention is to provide a composition and method that stimulates TNF-α production by cells of the immune system.

Another object of the present invention is to provide a composition that is simple to prepare.

Another object of the present invention is to provide a composition that is minimally toxic to the recipient.

These and other objects, features and advantages of the present invention will become apparent after a review of the following detailed description of the disclosed embodiment and the appended claims.

### BRIEF DESCRIPTION OF THE FIGURES

FIG. 1. Fluorescence [Fluo-3-AM] of Jurkat human T leukemia cells incubated with 0 µg/ml and 100 µg/ml of 6 base GT SEQ ID NO:25 (comparison).

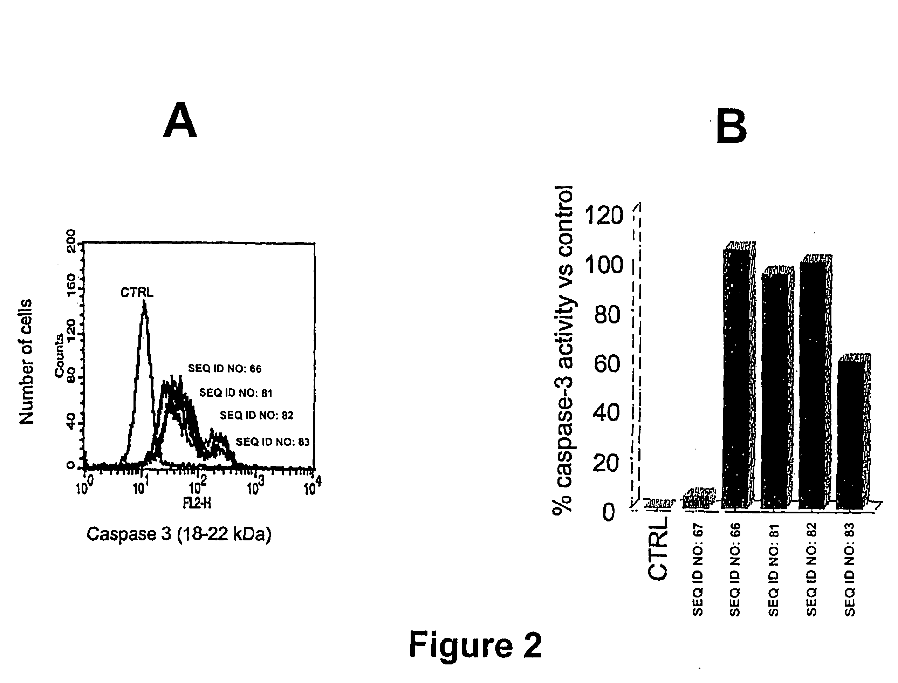

FIG. 2. Caspase 3 activation in Jurkat human T cell leukemia cells incubated without 0 µg/ml and 100 µg/ml of GT SEQ ID NOs:66, 67, 81, 82 and 83 measured cytometrically (A) and colorimetrically (B) (comparison).

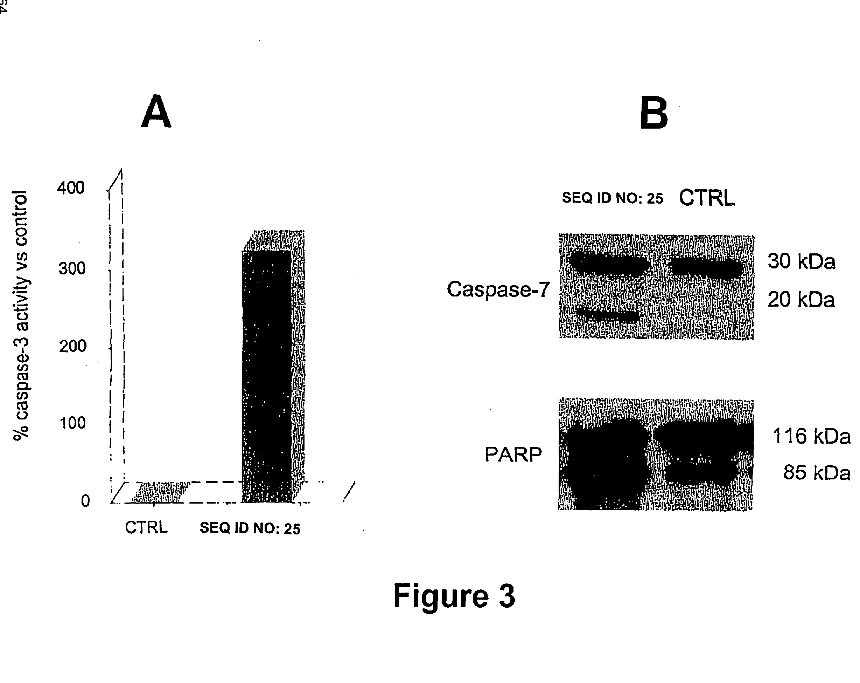

FIG. 3. Caspase activation in Jurkat human T cell leukemia cells. (A) Caspase 3 activation in cells incubated with 0 µg/ml and 100 µg/ml of 6 base GT SEQ ID NO:25; (B) Caspase 7 activation(a) and PARP content (b) in cells incubated with 0 µg/ml and 100 µg/ml of 6 base GT SEQ ID NO:25. (comparison).

### DETAILED DESCRIPTION OF THE INVENTION

The present invention provides a composition comprising a 3'-OH, 5'OH synthetic phosphodiester nucleotide sequence of SEQ ID NO:45: gggagg
wherein the sequence induces a response selected from the group consisting of induction of cell cycle arrest, inhibition of proliferation, activation of caspases and induction of apoptosis in cancer cells and production of cytokines by immune system cells.
A composition comprising the sequence SEQ ID NO:45 and a pharmaceutically acceptable carrier is administered to an animal, including a human, having cancer in an amount effective to treat the cancer in the animal, including the human. The unexpected and surprising ability of the sequence to induce cell cycle arrest, inhibit proliferation, induce apoptosis and activate caspases in cancer cells and to stimulate cytokine production by immune system cells addresses a long felt unfulfilled need in the medical arts and provides an important benefit for animals, including humans.

As used herein the word "sequence" refers to a 2 to 20 base 3'-OH, 5'-OH synthetic oligonucleotide comprising A, C, G and T bases.

As used herein, the abbreviations "GT", "AG" "CG", "GG", "AGT" and "CGT" refer to sequences comprising the named bases synthesized in any order.

As used herein, the word "response" refers to induction of cell cycle arrest, inhibition of proliferation, activation of caspases and induction of apoptosis, in cancer cells and stimulation of cytokine production by immune system cells.

As used herein, the phrases "therapeutic treatment" and "amount effective to" refer to an amount of a sequence effective to induce cell cycle arrest, inhibit proliferation, activate caspses and induce apoptosis in cancer cells and stimulate cytokine production by immune system cells.

As used herein, the phrases "suspension tumor model" and "solid tumor models" refer to primary or secondary carcinomas or sarcomas".

As used herein, the phrase "chemotherapeutic" is any agent approved by a regulatory agency of a country or a state government or listed in the U.S. Pharmacopoeia or other generally recognized pharmacopoeia to treat cancer in an animal, including a human.

As used herein, the word "antineoplastic" refers to preventing the development, maturation, proliferation or spread of cancer cells.

As used herein, the word "potentiates" refers to a degree of synergism that is greater than the additive activity of each agent.

As used herein, the word "synergism" refers to the coordinated action of two or more agents.
Administration of an effective amount of sequence SEQ ID NO:45 of the present invention to an animal, including a human, is a therapeutic treatment that prevents, treats or eliminates a disease including, but not limited to, cancer, rheumatoid arthritis, lympho-proliferative disorders and asthma. Cancers include, but are not limited to, squamous cell carcinoma, fibrosarcoma, sarcoid carcinoma, melanoma, mammary cancer, lung cancer, colorectal cancer, renal cancer, osteosarcoma, cutaneous melanoma, basal cell carcinoma, pancreatic cancer, bladder cancer, brain cancer, ovarian cancer, prostate cancer, leukemia, lymphoma and metastases derived therefrom.

The therapeutic effectiveness of a sequence may be increased by methods including, but not limited to, chemically modifying the base, sugar or phosphate backbone, chemically supplementing or biotechnologically amplifying the sequences using bacterial plasmids containing the appropriate sequences, complexing the sequences to biological or chemical carriers or coupling the sequences to tissue-type or cell-type directed ligands or antibodies

Compositions comprising the sequence SEQ ID NO:45 and a pharmaceutically acceptable carrier are prepared by uniformly and intimately bringing into association the sequence and the pharmaceutically acceptable carrier. Pharmaceutically acceptable carriers include liquid carriers, solid carriers or both. Liquid carriers are aqueous carriers, non-aqueous carriers or both and include, but are not limited to, aqueous suspensions, oil emulsions, water in oil emulsions, water-in-oil-in-water emulsions, site-specific emulsions, long-residence emulsions, sticky-emulsions, microemulsions and nanoemulsions. Solid carriers are biological carriers, chemical carriers or both and include, but are not limited to, viral vector systems, particles, microparticles, nanoparticles, microspheres, nanospheres, minipumps, bacterial cell wall extracts and biodegradable or non-biodegradable natural or synthetic polymers that allow for sustained release of the sequences. Methods used to complex a sequence to a solid carrier include, but are not limited to, direct adsorption to the surface of the solid carrier; covalent coupling to the surface of the solid carrier, either directly or via a linking moiety; and covalent coupling to the polymer used to make the solid carrier. Optionally, a sequence can be stabilized by the addition of non-ionic or ionic polymers such as polyoxyethylenesorbitan monooleates (Tweens) or hyaluronic acid.

Preferred aqueous carriers include, but are not limited to, water, saline and pharmaceutically acceptable buffers. Preferred non-aqueous carriers include, but are not limited to, a mineral oil and a neutral oil and mixtures thereof. Optionally, excipients may be included regardless of the pharmaceutically acceptable carrier used to present the sequence to the responding cells. These excipients include, but are not limited to, antioxidants, buffers, and bacteriostats, and may include suspending agents and thickening agents.

Sequence SEQ ID NO:45 may be administered alone, or in combination with other therapeutic modalities including, but not limited to, chemotherapeutic agents, immunotherapeutic agents, antimicrobial agents, antiviral agents or in combination with radiation therapy. Chemotherapeutic agents include, but are not limited to, anti-metabolites, DNA damaging, microtubule destabilizing, microtubule stabilizing, actin depolymerizing, growth inhibiting, topoisomerase inhibiting, HMG-CoA inhibiting, purine inhibiting, pyrimidine inhibiting, metaloproteinase inhibiting, CDK inhibiting, angiogenesis inhibiting and differentiation enhancing agents.

Routes of administration include, but are not limited to, oral, topical, subcutaneous, transdermal, subdermal, intra-muscular, intra-peritoneal, intra-vesical, intra-articular, intra-arterial, intra-venous, intra-dermal, intra-cranial, intra-lesional, intra-tumoral, intra-ocular, intra-pulmonary, intra-spinal, intra-prostatic, placement within cavities of the body, nasal inhalation, pulmonary inhalation, impression into skin and electrocorporation.

Depending on the route of administration, the volume per dose is preferably about 0.001 to 100 ml per dose, more preferably about 0.01 to 50 ml per dose and most preferably about 0.1 to 30 ml per dose. Preferably, the amount of sequence administered per dose is from about 0.001 to 100 mg/kg, more preferably from about 0.01 to 10 mg/kg and most preferably from about 0.1 to 5 mg/kg. The sequence SEQ ID NO:45 or sequence plus a therapeutic agent can be administered in a single dose treatment, in multiple dose treatments or continuously infused on a schedule and over a period of time appropriate to the disease being treated, the condition of the recipient and the route of administration. Moreover, the sequence can be administered before, at the same time as, or after administration of the therapeutic agent.

The sequence SEQ ID NO:45 in combination with chemotherapeutic agent is administered to an animal having cancer in an amount effective to potentiate the anti-neoplastic effect of the chemotherapeutic agent. Preferably, the amount of therapeutic agent administered per dose is from about 0.001 to 1000 mg/m² or from about 0.01 to 1000 mg/kg, more preferably from about 0.01 to 500 mg/m² or from about 0.01 to 500 mg/kg and most preferably from about 0.1 to 100 mg/m² or from about 0.1 to 100 mg/kg. The particular sequence and the particular therapeutic agent administered, the amount per dose, the dose schedule and the route of administration should be decided by the practitioner using methods known to those skilled in the art and will depend on the type of cancer, the severity of the cancer, the location of the cancer and other clinical factors such as the size, weight and physical condition of the recipient. In addition, *in vitro* assays may optionally be employed to help identify optimal ranges for sequence administration and for sequence plus therapeutic agent administration.

Although not wanting to be bound by the following hypothesis, it is thought that the sequence SEQ ID NO:45 of the present invention belongs to a new family of structures and that it does not function as antisense RNA, DNA, triple helix forming DNA, telomerase inhibitor, transcription activator or transcription inhibitor.

The following examples will serve to further illustrate the present invention without, at the same time, however, constituting any limitation thereof.

### Example 1

### Preparation of sequences

Phosphodiester and phosphorothioate sequences were prepared by HUKABEL Scientific Ltd, (Montréal, Québec, Canada) using the EXPEDITE^{™} 8900 automated DNA synthesis system (PersSeptive Biosystems, Inc., Farminghan, MA) and by Sigma-Genosys (Woodlands, TX) using Abacus Segmented Synthesis Technology. Unless stated otherwise, the sequences used were phosphodiester sequences. Unless stated otherwise, immediately prior to use, the sequences were dispersed in autoclaved deionized water or in an autoclaved pharmaceutically acceptable buffer such as, but not limited to, saline.

### Example 2

### Cells and reagents

All cell lines were obtained from the American Type Culture Collection (ATCC, Rockville, MD) and were cultured in the medium recommended by the ATCC. Table 1 shows the cell lines, their origins and their properties.

**Table 1**

| Cell lines | | |
|---|---|---|
| CELL LINE | ORIGIN | PROPERTIES |
| THP-1 | Human acute monocytic leukemia | Suspension tumor model p53 null |
| MCF-7 | Human breast cancer | Solid tumor model; non-metastatic Caspase 3-negative; estrogen-dependent |
| JURKAT | Human T cell leukemia | Suspension tumor model Atypical multi-drug resistance associated with p190-MRP protein |
| PC-3 | Human prostate cancer | Solid tumor model; metastatic p53 mutated; androgen-independent (hormone refractory) |
| LNCaP | Human prostate cancer | Solid tumor model; metastatic TGF-beta I receptor-negative; androgen-dependent |
| OVCAR-3 | Human ovarian cancer | Solid tumor model; metastatic p53 mutated; p21/waf 1/Cip-1 deleted |
| SK-OV-3 | Human ovarian cancer | Solid tumor model; metastatic p53 deleted; p21/waf-1/Cip deleted; p15^{ink4B}, p16^{ink4} deleted |
| HL-60 | Human promyelocytic leukemia | Suspension tumor model p53 mutated |
| EL-4 | Murine T lymphoma | Suspension tumor model |
| A20 | Murine B cell leukemia | Suspension tumor model |
| L-1210 | Murine leukemia | Suspension tumor model |
| D-17 | Canine osteosarcoma | Solid tumor model |
| CF-51 | Canine mammary gland cancer | Solid tumor model |

Cells were seeded in 6 (1 ml/well), 24 (0.5 ml/well) or 96 (0.1 ml/well) well flat-bottom microplates and were maintained at 37°C in a 5% CO₂ atmosphere. Unless stated otherwise, 2.5 X 10⁵ cells/ml were incubated with 0 µg/ml (control) and 100 µg/ml (treated) of 2 to 45 base sequences containing A, C, G and T for 48 h.

### Example 3

### Measurement of cell proliferation

Cell proliferation was measured using dimethylthiazol-diphenyltetrazolium (MTT) reduction (Mosman et al. J. Immunol. Methods 65:55, 1983). MTT was measured at a wavelength of 570 nm using a multiplate spectrophotometer reader (ELX800, Bio-TEK Instruments Inc., Winooski, VT).

### Example 4

Jurkat T cells were incubated with 6 base GT, AG, CG, GG, AGT and CGT sequences (Table 2).

**Table 2**

| % inhibition Jurkat human leukemia T cell proliferation | |
|---|---|
| SEQUENCE | % INHIBITION |
| TGTGTG-(TG)₃ | 36 |
| SEQ ID NO:9-(6 bases) | |
| GTGTGT-(GT)₃ | 48 |
| SEQ ID NO:10-(6 bases) | |
| TTTGTT-TT(TG)₁TT | 31 |
| SEQ ID NO:23-(6 bases) | |
| GGTGGG-GG(TG)₁GG | 48 |
| SEQ ID NO:24-(6 bases) | |
| GGGTGG-GG(GT)₁GG | 60 |
| SEQ ID N0:25-(6 bases) | |
| TTGTTT-TT(GT)₁TT | 34 |
| SEQ ID N0:26-(6 bases) | |
| AAGTAA-AA(GT)₁AA | 13 |
| SEQ ID N0:27-(6 bases) | |
| CCGTCC-CC(GT)₁CC | 11 |
| SEQ ID NO:28-(6 bases) | |
| TGGTTG-TG(GT)₁TG | 42 |
| SEQ ID NO:29-(6 bases) | |
| ATGTAT-AT(GT)₁ AT | 16 |
| SEQ ID NO:30-(6 bases) | |
| AGGTGA-AG(GT)₁GA | 10 |
| SEQ ID NO:31-(6 bases) | |
| GAGTGA-GA(GT)₁GA | 24 |
| SEQ ID NO:32-(6 bases) | |
| GGGTCT-GG(GT)₁CT | 15 |
| SEQ ID NO:33-(6 bases) | |
| CCGTGG-CC(GT)₁GG | 37 |
| SEQ ID NO:34 -(6 bases) | |
| GGGTCC-GG(GT)₁CC | 20 |
| SEQ ID NO:35-(6 bases) | |
| CTGTCT-CT(GT)₁CT | 19 |
| SEQ ID NO:36-(6 bases) | |
| TCGTTC-TC(GT)₁TC | 20 |
| SEQ ID NO:37-(6 bases) | |
| CGCTGC-CG(GT)₁GC | 16 |
| SEQ ID NO: 38-(6 bases) | |
| TTGTGG-TT(GT)₁GG | 35 |
| SEQ ID NO:39-(6 bases) | |
| GGGTTT-GG(GT)₁TT | 31 |
| SEQ ID NO:40-(6 bases) | |
| GGTTGG-GG(TT)₁GG | 43 |
| SEQ ID NO:41-(6 bases) | |
| GGAAGG-GG(AA)₁GC | 22 |
| SEQ ID N0:42-(6 bases) | |
| GGCCGG-GG(CC)GG | 29 |
| SEQ ID NO:43-(6 bases) | |
| GGGGGG-GG(GG)₁GG | 26 |
| SEQ ID NO:44 -(6 bases) | |
| GGGAGG-GG(GA)₁GG (invention) | 28 |
| SEQ ID NO:45-(6 bases) | |
| GGGCGG-GG(GC)₁GG | 23 |
| SEQ ID NO:46-(6 bases) | |
| GGAGGG-GG(AG)₁GG | 14 |
| SEQ ID NO:47 - (6 bases) | |
| GTGGGG-(GT)₁G₄ | 26 |
| SEQ ID NO:48 - (6 bases) | |
| TTAGGG-TT(AG)₁GG | 45 |
| SEQ ID NO:49-(6 bases) | |

As shown in Table 2, 6 base GT sequences inhibited Jurkat T cell proliferation. GT SEQ ID NO:25 inhibited proliferation 60% and AGT SEQ ID NO:49 inhibited proliferation 45%. Comparison of the relative potency of GT SEQ ID NO:25 and AGT SEQ ID NO:49 using PHARMIPCS-4 Software (Microcomputer Specialists, Philadelphia, PA) showed the potency of GT SEQ ID NO:25 to be 3.4 times that of AGT SEQ ID NO:49. AGT SEQ ID NO:49-phosphorothioate is reported to inhibit telomerase activity and to induce apoptosis in Burkitt lymphoma cells (Mata et al. Toxicol. Appl. Pharmacol. 144:189,1997).

To determine telomerase activity, extracts from 2 x 10⁵ Jurkat T cells were assayed using the PCR-telomeric repeat amplification protocol (TRAP) (Roche, Laval, Quebec, Canada). At concentrations between 1 and 100 µg/ml, GT SEQ ID NO:25-phosphodiester showed between 0 and 10% anti-telomerase activity, whereas AGT SEQ ID NO:49-phosphorothioate showed between 30 and 75% anti-telomerase activity. Neither GT SEQ ID NO:25-phosphorothioate nor GT SEQ ID NO:49-phosphodiester showed any anti-telomerase actvity.

### Example 5 (comparison)

### Inhibition of cell proliferation by phosphodiester and phosphorothioate sequences

Modification of natural phosphodiester sequences by substitution of a sulfur atom for a nonbridging oxygen atom on one or more of the phosphate groups has been reported to increase the stability of oligonucleotide sequences to endonucleases in biological fluids and cells (Crooke et al. Anticancer Drugs 6:609, 1991).

Jurkat human leukemia T cells (Table 3), LNCaP human prostate cancer cells (5 x 10⁵ cells/ml) (Table 4) and MCF-7 human breast cancer cells (5x10⁵ cells/ml) (Table 5) were incubated with 6 base GT sequences, having either oxygen (phosphodiester) or sulfur (phosphorothioate) as a nonbridging atom on the phosphate group.

**Table 3**

| % inhibition of Jurkat human leukemia T cell proliferation | | |
|---|---|---|
| SEQUENCE | % INHIBITION | |
| | PHOSPHODIESTER | PHOSPHOROTTUOA-M |
| TGTGTG-(TG)₃ (6 bases) | 37 | -17 |
| SEQ ID NO:9 phosphodiester; phosphorothioate | | |
| GTGTGT-(GT)₃ (6 bases) | 44 | 0 |
| SEQ ID NO:10 phosphodiester; phosphorothioate | | |
| TTTGTT-TT(TG)₁TT (6 bases) | 31 | 4 |
| SEQ ID NO:23 phosphodiester; phosphorothioate | | |
| GGTGGG-GG(TG)₁GG (6 bases) | 48 | 6 |
| SEQ ID NO:24 phosphodiester; phosphorothioate | | |
| GGGTGG-GG(GI)₁GO (6 bases) | 60 | 0 |
| SEQ ID NO:25 phosphodiester; phosphorothioate | | |
| TTGTTT(GT)₁TT (6 bases) | 34 | 0 |
| SEQ ID NO:26 phosphodiester; phosphorothioate | | |

**Table 4**

| % inhibition of LNCaP human prostate cancer cell proliferation | | |
|---|---|---|
| SEQUENCE | % INHIBITION | |
| | PHOSPHODIESTER | PHOSPHOROTHIOATE |
| TGTGTG-(TG)₃ (6 bases) | -9 | -16 |
| SEQ ID NO:9 phosphodiester, phosphorothioate | | |
| GTGTGT-(GT)₃ (6 bases) | -4 | -20 |
| SEQ ID NO: 10 phosphodiester, phosphorothioate | | |
| TTTGTT-TT(TG)₁TT (6 bases) | 14 | -11 |
| SEQ ID NO:23 phosphodiester, phosphorothioate | | |
| GGTGGG-GG(TG)₁GG (6 bases) | 17 | -17 |
| SEQ ID NO:24 phosphodiester, phosphorothioate | | |
| GGGTGG-GG(GT)₁GG (6 bases) | 18 | -8 |
| SEQ ID NO:25 phosphodiester; phosphorothioate | | |
| TTGTTT-TT(GT)₁TT (6 bases) | 22 | -1 |
| SEQ ID NO:26 phosphodiester; phosphorothioate | | |

**Table 5**

| % inhibition of MCF-7 human breast cancer cell proliferation | | |
|---|---|---|
| SEQUENCE | % INHIBITION | |
| | PHOSPHODIESTER | PHOSPHOROTHIOATE |
| TGTGTG-(TG)₃ (6 bases) | 6 | 6 |
| SEQ ID NO:9 phosphodiester, phosphorothioate | | |
| GTGTGT-(GT)₃ (6 bases) | 31 | 12 |
| SEQ ID NO:10 phosphodiester; phosphorothioate | | |
| TTTGTT-TT(TG)₁TT (6 bases) | 7 | 8 |
| SEQ ID NO:23 phosphodiester; phosphorothioate | | |
| GGTGGG-GG(TG)₁GG (6 bases) | 41 | 12 |
| SEQ ID NO:24 phosphodiester; phosphorothioate | | |
| GGGTGG-GG(GT)₁GG (6 bases) | 41 | 12 |
| SEQ ID NO: 25 phosphodiester; phosphorothioate | | |
| TTGTTT-TT(GT)₁TT (6 bases) | 20 | 6 |
| SEQ ID NO:26 phosphodiester, phosphorothioate | | |

As shown in Tables 3, 4 and 5, 6 base GT-phosphodiester sequences inhibited Jurkat T, LNCaP and MCF-7 cell proliferation more effectively than 6 base GT-phosphorothioate sequences.

### Example 6 (comparison)

### Inhibition of cell proliferation by mixed phosphodiester and phosphorothioate sequences

Jurkat human leukemia T cells (Table 16) and MCF-7 human breast cancer cells (5 x 10⁵ cells/ml) (Table 17) were incubated with the 6 base GT SEQ ID NO:25, wherein either oxygen (phosphodiester) or sulfur (phosphorothioate) was the nonbridging atom on the phosphate group.

**Table 6**

| % inhibition of Jurkat human leukemia T cell proliferation | |
|---|---|
| SEQUENCE* | % INHIBITION |
| GₒGₒGₒTₒGₒGₒ-GₒGₒ(GₒTₒ)₁GₒGₒ (oxygen atom I to 6) | 60 |
| SEQ ID NO: 25-(6 bases) | |
| GₒGₒGₛTₒGₒGₒ-GₒGₒ(GₛTₒ)₁GₒGₒ (oxygen atom 1,2,4,5,6; sulfur atom 3) | 17 |
| SEQ ID NO: 25-(6 bases) | |
| GₒGₒGₒTₛGₒGₒ-GₒGₒ(GₒTₛ)₁GₒGₒ (oxygen atom 1,2,3,5,6; sulfur atom 4) | 12 |
| SEQ ID NO: 25-(6 bases) | |
| GₒGₒGₛTₛGₒGₒ-GₒGₒ(GₛTₛ)₁GₒGₒ (oxygen atom 1,2,5,6; sulfur atom 3,4) | 13 |
| SEQ ID NO: 25-(6 bases) | |
| GₛGₒGₒTₒGₒGₛ-GₛGₒ(GₒTₒ)₁GₒGₛ (oxygen atom 2,3,4,5; sulfur atom 1,6) | 16 |
| SEQ ID NO:25-(6 bases) | |
| GₒGₛGₒTₒGₛGₒ-GₒGₛ(GₒTₒ)₁GₛGₒ (oxygen atom 1,3,4,6; sulfur atom 2,5) | 11 |
| SEQ ID NO:25-(6 bases) | |
| GₛGₛGₒTₒGₛGₛ-GₛGₛ(GₒTₒ)₁GₛGₛ (oxygen atom 3,4; sulfur atom 1,2,5,6) | - 13 |
| SEQ ID NO:25-(6 bases) | |
| G₅G₅G₅T₅G₅G₅-G₅G₅(G₅T₅)₁G₅G₅ (sulfur atom 1 to 6) | 0 |
| SEQ ID NO:25 -(6 bases; phosphorothioate) | |

| | |
|---|---|
| *Note: "o" represents an oxygen atom and "s" represents a sulfur atom on the phosphate group | |

**Table 7**

| % inhibition of MCF-7 human breast cancer cell proliferation | |
|---|---|
| SEQUENCE* | % INHIBITION |
| GₒGₒGₒTₒGₒGₒ-GₒGₒ(GₒTₒ)₁GₒGₒ (oxygen atom I to 6) | 41 |
| SEQ ID NO:25-(6 bases; phosphodiester) | |
| GₒGₒGₛTₒGₒGₒ-GₒGₒ(GₛTₒ)₁GₒGₒ (oxygen atom 1,2,4,5,6; sulfur atom 3) | 12 |
| SEQ ID NO:25-(6 bases) | |
| GₒGₒGₒTₛGₒGₒ-GₒGₒ(GₒTₛ)₁GₒGₒ (oxygen atom 1,2,3,5,6; sulfur atom 4) | 0 |
| SEQ ID NO:25-(6 bases) | |
| GₒGₒGₛTₛGₒGₒ-GₒGₒ(GₛTₛ)₁GₒGₒ (oxygen atom 1,2,5,6; sulfur atom 3,4) | 43 |
| SEQ ID NO:25-(6 bases) | |
| GₛGₒGₒTₒGₒGₛ-G_{g}Gₒ(GₒTₒ)₁GₒGₛ (oxygen atom 2,3.4,5; sulfur atom 1,6) | 12 |
| SEQ ID NO:25-(6 bases) | |
| GₒGₛGₒTₒGₛGₒ-GₒGₛ(GₒTₒ)₁GₛGₒ (oxygen atom 1,3,4,6; sulfur atom 2,5) | 13 |
| SEQ ID NO:25-(6 bases) | |
| GₛGₛGₒTₒGₛGₛ-GₛGₛ(GₒTₒ)₁GₛGₛ (oxygen atom 3.4; sulfur atom 1,2,5,6) | -3 |
| SEQ ID NO 25-(6 bases) | |
| GₛGₛGₛTₛGₛGₛ-GₛGₛ(GₛTₛ)₁GₛGₛ; (sulfur atom 1 to 6) | 12 |
| SEQ ID NO:25 (6 bases; phosphorothioate) | |

| | |
|---|---|
| *Note: "o" represents an oxygen atom and "s" represents a sulfur atom on the phosphate group. | |

As shown in Tables, 6 and 7, substitution of a sulfur atom for a nonbridging oxygen atom on one or more phosphate groups of 6 base GT SEQ ID NO: 25 resulted in a significant decrease in inhibition of Jurkat T and MCF-7 cell proliferation.

### Example 7 (comparison)

### Induction of cell cycle arrest

Cell cycle stage was determined using a CYCLETEST^{™} PLUS DNA commercial kit (Becton Dickinson). Briefly, nuclei from cells were obtained by dissolving the cell membrane in a nonionic detergent, eliminating the cell cytoskeleton and nuclear proteins with trypsin, digesting the cellular RNA with RNase and stabilizing the nuclear chromatin with spermine. Propidium iodide was added to the cell nuclei and their fluorescence was analyzed in a flow cytometer equipped with electronic doublet discrimination capability (FACSCalibur, Becton Dickinson, San Jose, CA). Accumulation of cells in G₀/G1, early S (SE), mid S (SM), late S (SL) or G₂/M phases of the cell cycle was analyzed using MODFIT LT software (Verity Software House Inc., Topsham, MA).

Exponentially growing Jurkat human leukemia T cells (Table 8) and MCF-7 human breast cancer cells (5 × 10⁵ cells/ml) (Table 9) were incubated for 24 h with 2, 6, 15, 18, 27 and 45 base sequences containing A, C, G and T. The cells were collected and centrifuged and cell cycle stage was determined.

**Table 8**

| Induction of cell cycle arrest in Jurkat T human leukemia cells | | | | | | |
|---|---|---|---|---|---|---|
| | % of cells in phase: | | | | | |
| | G₀/G₁ | SE | SM | SL | G₂/M | Arrest* |
| Untreated cells | 31.4 | 19.1 | 14.3 | 11.6 | 23.6 | None |
| GG(GT)₁GG | 28.5 | 46.3 | 14.6 | 10.7 | 0.0 | End SE |
| SEQ ID NO:25-(6 bases) | | | | | | |
| TT(GT)₁TT | 32.6 | 11.5 | 12.8 | 10.7 | 32.4 | End |
| SEQ ID NO:26 bases) | | | | | | G₂/M (weak) |
| GT(GT)₁GT | 30.8 | 41.9 | 16.8 | 102 | 03 | End SE |
| SEQ ID NO:10-(6 bases) | | | | | | |
| AG(GT)₁GA | 35.2 | 29.1 | 10.4 | 82 | 17.1 | Mid SE |
| SEQ ID NO:31-(6 bases) | | | | | | |
| GG(AA)₁GG | 48.0 | 19.8 | 8.5 | 5.8 | 34.1 | End |
| SEQ ID NO:42-(6 bases) | | | | | | G₀/G₁ |
| GG(CC)₁GG | 26.5 | 21.3 | 22.8 | 10.7 | 18.7 | End SM |
| SEQ ID NO:43 (6 bases) | | | | | | (weak) |
| GG(GT)₁GG | 34.9 | 14.8 | 15.0 | 10.6 | 24.7 | None |
| SEQ ID NO:25-(6 bases phosphorothioate) | | | | | | |
| (G₁T)₁₃G | 40.6 | 35.6 | 14.2 | 9.3 | 0.3 | End SE |
| SEQ ID NO: 1-(27 bases) | | | | | | |
| (G₁T)₁₃G | 33.7 | 17.6 | 13.2 | 11.0 | 24.5 | None |
| SEQ ID NO: 1-(27 bases phosphorothioate) | | | | | | |
| (G₃T)₆G₃ | 34.3 | I5.5 | 13.6 | 10.3 | 26.4 | None |
| SEQ. ID NO:2-(27 bases) | | | | | | |
| (G₅T)₄G₃ | 40.5 | 133 | 12.9 | 9.7 | 23.6 | None |
| SEQ ID NO:3-(27 bases) | | | | | | |
| (G₇T)₃G₃ | 36.5 | 16.3 | 13.8 | 11.1 | 223 | None |
| SEQ ID NO:4-(27 bases) | | | | | | |
| AACCACAAGCCCAAC | 39.6 | 13.5 | 12.8 | .9.5 | 24.6 | None |
| SEQ ID NO:67-(15 bases) | | | | | | |
| TT(AG)₁GG | 24.6 | 37.2 | 19.5 | 5.9 | 12.8 | Mid SM |
| SEQ ID NO:49-(6 bases) | | | | | | |
| (GT)₉ | 24.2 | 26.7 | 24.0 | 8.7 | 16.4 | Mid SM |
| SEQ ID NO:18-(18 bases) | | | | | | |
| BCG A-1 | 19.8 | 31.7 | 22.5 | 14.0 | 12.0 | Mid SM |
| SEQ ID NO:71-(45 bases) | | | | | | |
| BGC A-3 | 32.3 | 20.2 | 14.1 | 12.0 | 21.4 | None |
| SEQ ID NO:73-(45 bases) | | | | | | |
| TG | 23.1 | 52.3 | 14.8 | 9.8 | 0.0 | End SE |
| SEQ ID NO:51-(2 bases) | | | | | | |

As shown in Table 8, in Jurkat T cells, 2, 6 and 27 base GT sequences induced arrest in the SE phase of the cell cycle, 6 base CG and AGT, 18 base GT and 45 base BCG A-1 sequences induced arrest in the SM phase of the cell cycle and a 6 base AG sequence induced arrest in the G₀/G₁ phase of the cell cycle.

**Table 9**

| Induction of cell cycle arrest in MCF-7 human breast cancer cells | | | | | | |
|---|---|---|---|---|---|---|
| | % cells in phase: | | | | | |
| | G₀/G₁ | SE | SM | SL | G₂/M | Arrest* |
| Untreated cells | 23.6 | 14.4 | 10.8 | 11.1 | 40.1 | None |
| GG(GT)₁GG | 21.9 | 27.6 | 222 | 10.9 | 17.4 | End SM |
| SEQ ID NO:25-(6 bases) | | | | | | |
| Tr(AG)₁GG | 20.0 | 18.6 | 25.7 | 20.7 | 15.0 | Mid SM |
| SEQ ID NO:49-(6 bases) | | | | | | |
| (GT)₉ | 25.3 | 31.6 | 16.9 | 10.5 | 15.7 | Mid SM |
| SEQ ID NO:18-(18 bases) | | | | | | |
| TG | 172 | 36.4 | 13.4 | 14.1 | 17.9 | End SE |
| SEQ ID NO: 51-(2 bases) | | | | | | |

As shown in Table 29, in MCF-7 cells, 2 and 6 base GT sequences induced arrest in the SE phase of the cell cycle, a 6 base AGT sequence and an 18 base GT sequence induced arrest in the SM phase of the cell cycle.

### Example 8 (comparison)

### Induction of cell cycle arrest by GT SEQ ID NO.- 25, AC SEQ ID NO: 79 and GT SEQ ID NO: 25 + AC SEQ ID NO: 79

Jurkat human cell leukemia T cells (1 X 10⁶ cells/ml) were incubated for 24 h with 6 base GT SEQ ID NO:25, complementary 6 base AC SEQ ID NO:79 and 6 base GT SEQ ID NO:25 + 6 base AC SEQ ID NO:79. GT SEQ ID NO:25 and AC SEQ ID NO:79 were hybridized by mixing the oligonucleotides (1:1) and heating for 10 minutes at 65°C. As controls, GT SEQ ID NO:25 and AC SEQ ID NO:79 were heated for 10 minutes at 65°C (Table 30).

**Table 10**

| Induction of cell cycle arrest in Jurkat human leukemia T cells | | | | | | |
|---|---|---|---|---|---|---|
| | % cells in phase: | | | | | |
| | G₀/G₁ | SE | SM | SL | G₂/M | Arrest |
| Untreated cells | 31.7 | 152 | 13.7 | 14.0 | 25.4 | None |
| GG(GT)₁GG | 28.0 | 45.8 | 14.0 | 11.3 | 0.9 | End SE |
| SEQ ID NO:25-(6 bases) | | | | | | |
| CC(AC)₁CC | 36.0 | 10.4 | 13.4 | 9.7 | 30.5 | None |
| SEQ ID NO:79-(6 bases) | | | | | | |
| GT(GT)₁GT+CC(AC)₁CC None SEQ NO: 25 + SEQ NO: 79-(12 bases) | 35.0 | 13.0 | 10.1 | 8.7 | 33.2 | None |

As shown in Table 30, 6 base GT SEQ ID NO:25 induced arrest at the end of the SE phase of the cell cycle, whereas the complementary 6 base AC SEQ ID NO:79 had no effect on the cell cycle. Hybridization of GT SEQ ID NO:25 and AC SEQ ID NO:79 neutralized GT SEQ ID NO:25 induction of cell cycle arrest. These data demonstrate that to be effective, the sequences of the present invention must be single stranded.

### Example 9

### Induction of apoptosis

Redistribution of plasma membrane phosphatidylserine and release of nuclear matrix protein (NuMA) are characterisrics of cells undergoing apoptosis (Martin et al. J. Exp. Med., 182:1545, 1995; Miller et al. Biotechniques, 15:1042, 1993).

The redistribution of phosphatidylserine in the plasma membrane during apoptosis was measured by flow cytometry using FITC-conjugated annexin V (BD Pharmingen, San Diego, CA). NuMA release into the supernatant was determined using a commercial ELISA kit (Oncogen/Calbiochem, Cambridge, MA).

Jurkat human leukemia T cells were incubated with 50 µM of 3, 4, 5, 6 and 7 GT base sequences, a 5 base ACGT sequence, 6 base AG, GG, AGT and CGT sequences and a 7 base GG sequence. Table 31 shows % of cells in apoptosis (positive for phosphatidyl-serine/annexin V staining (PS/A-V)) and % NuMA released from the cells.

**Table 11**

| Induction of apoptosis in Jurkat T cell leukemia cells | | |
|---|---|---|
| SEQUENCE | % of cells in apoptosis (positive for PS/A-V staining) | % NuMA released (treated vs untreated cells) |
| Untreated cells | 4 | 0 |
| GG(GT)₁GG | 27 | 69 |
| SEQ ID NO:2-(6 bases) | | |
| GG(GA)₁GG (invention) | 27 | 74 |
| SEQ ID NO:45-(6 bases) | | |
| GG(GC)₁GG | 16 | 11 |
| SEQ ID NO:46-(6 bases) | | |
| GG(GG)₁GG | 5 | 0 |
| SEQ ID NO:44-(6 bases) | | |
| AA(GT)₁AA | 20 | 56 |
| SEQ ID NO:27-(6 bases) | | |
| CC(GT)₁CC | 6 | 0 |
| SEQ ID NO:28- (6 bases) | | |
| TT(GT)₁TT | 14 | 23 |
| SEQ ID NO:26-(6 bases) | | |
| GT(GT)₁GT | 33 | 90 |
| SEQ ID NO:10-(6 bases) | | |
| GG(GT)₁ | 21 | 64 |
| SEQ ID N0:78-(4 bases) | | |
| (GT)₁GG | 24 | 60 |
| SEQ ID N0:52-(4 bases) | | |
| G(GT)₁G | 24 | 112 |
| SEQ ID N0:56-(4 bases) | | |
| (GT)₁G | 21 | 97 |
| SEQ ID N0:8-(3 bases) | | |
| T(GT)₁ | 10 | 35 |
| SEQ ID NO:7-(3 bases) | | |
| GG(GT)₁G | 25 | 92 |
| SEQ ID NO:6- (5 bases) | | |
| G(GT)₁GG | 25 | 120 |
| SEQ ID NO:60-(5 bases) | | |
| GG(GG)₁GGG | 12 | 26 |
| SEQ ID NO:63-(7 bases) | | |
| GGG(GT)₁GG | 30 | 123 |
| SEQ ID NO:62-(7 bases) | | |
| CG(GT)₁A | 6 | 9 |
| SEQ ID NO:80-(5 bases) | | |

As shown in Table 31, 3, 4, 5, and 6 base GT, AG, CG and AGT sequences induced apoptosis of Jurkat T cells. Five base ACGT and 6 base CGT and GG sequences did not induce apoptosis of Jurkat T cells.

### Example 10 (comparison)

### Increase in intracellular calcium (Ca²⁺)_{f}

Increases in intracellular calcium (Ca²⁺)ᵢ are reported to be associated with apoptosis induction (Lam et al. Mol. Endocrinol. 7:686, 1993). (Ca²⁺)ᵢ was followed using the fluorescent probe Fluo-3-AM (Cell Permaant, Molecular Probes, Inc., Eugene, OR). An increase in Fluo-3-AM fluorescence is indicative of an increase in (Ca²⁺)ᵢ.

Jurkat human leukemia T cells were incubated for 24 h with 6 base GT SEQ. NO:25. Cells were collected by centrifugation, suspended in PBS containing 1% FBS and loaded with 10 µM Fluo-3-AM for 1 h at 37°C. Cell fluorescence was measured at 488 nm excitation and 530 nm emission (FL1 detector). Data were analyzed on a FACSCALIBUR using the program CellQUEST (Becton Dickinson).

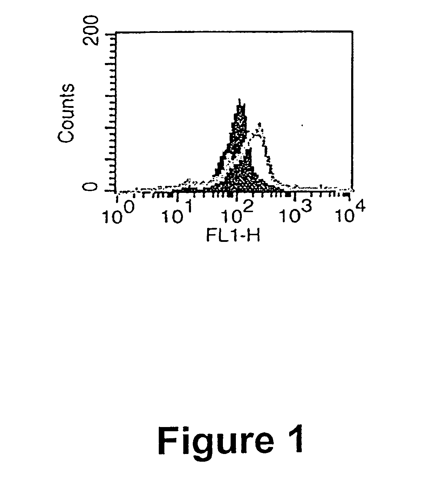

As shown in Figure 1, incubation of Jurkat T cells with 6 base GT SEQ ID NO:25 caused an 88% increase in cell fluorescence, indicative of an increase in (Ca²⁺)ᵢ.

### Example 11

### Induction of cytokine production

Unless stated otherwise, 1 x 10⁶ cells were incubated with 100 µg/ml of each of the sequences tested for 48 h at 37°C in 5% CO₂. Production of cytokines IL-6, IL-10, IL-12, IL-1beta and TNF-alpha was determined in pg/ml in 100 µl of culture supernatant using the appropriate commercial ELISA (BioSource, Camarillo CA). The IL-12 ELISA measures both IL-12 p70 complex and free p40 subunit. Results are expressed as the "fold" (x) increase in cytokine production by treated cells compared to control cells.

THP-1 human acute monocytic leukemia cells were incubated with 2, 3, 6, 9, 12, 14, 15 and 18 base GT sequences and production of the cyotkine IL-6 was determined (Table 35).

**Table 12**

| Cytokine production by TNP-1 human acute monocytic leukemia cells | |
|---|---|
| SEQUENCE | ↑ IL-6 |
| TG-(TG)₁T SEQ ID NO:7-(3 bases) | 2.7 x |
| TG-(TG)₁ SEQ ID NO:51-(2 bases) | 2.9 x |
| TGTGTG-(TG)₃ SEQ ID NO:9-(6 bases) | 4.0 x |
| GTGTGT-(GT)₃ SEQ ID NO:10-(6 bases) | 5.0 x |
| TGTGTGTG-(TG)₄T SEQ ID NO:11-(9 bases) | 5.4 x |
| GTGTGTGTG-(GT)₄G SEQ ID NO:12-(9 bases) | 5.4 x |
| TGTGTGTGTGT-(TG)₆ SEQ ID NO:13-(12 bases) | 5.7 x |
| GTGTGTGTGTGT-(GT)₆ SEQ ID NO:14-(12 bases) | 1.3 x |
| TGTGTGTGTGTGT-(TG)₇ SEQ ID NO:15-(14 bases) | 1.0 x |
| GTGTGTGTGTGTGTG-(GT)₇G SEQ ID NO:16-(15 bases) | 2.6 x |
| TGTGTGTGTGTGTGTGT-(TG)₉ SEQ ID NO:17-(18 bases) | 2.2 x |
| GTGTGTGTGTGTGTGTGT-(GT)₉ SEQ ID NO:18-(18 bases) | 2.8 x |

As shown in Table 35, 2, 3, 6, 9, 12, 14, 15 and 18 base GT sequences increased THP-1 cell production of the cytokine IL-6.

THP-1 human acute monocytic leukemia cells were incubated with 6 base GT, AG, CG, GG, AGT and CGT sequences and production of the cytokines IL-12 and IL-6 was determined (Table 13).

**Table 13**

| Cytokine production by THP-1 human acute monocytic leukemia cells | | |
|---|---|---|
| SEQUENCE | ↑ IL-12 | ↑ IL-6 |
| TGTGTG-(TG)₃ SEQ ID NO:9 | 1.8x | 4.0x |
| GTGTGT-(GT)₁ SEQ ID NO:10 | 22x | 5.0x |
| TTTGTT-TT(TG)₁TT SEQ ID NO:23 | 3.5x | 4.9x |
| GGTGGG-GG(TG)₁GG SEQ ID NO:24 | 3.7x | 6.9x |
| GGGTGG-GG(GT)₁GG SEQ ID NO:25 | 2.3x | 3.1x |
| TTGTTT-TT(GT)₁TT SEQ ID NO:26 | 3.5x | 5.3 |
| AAGTAA-AA(GT)₁AA SEQ ID NO:27 | 6.0x | 12.8x |
| CCGTCC-CC(GT)₁CC SEQ ID NO:28 | 3.8x | 12.6x |
| TGGTTG -TG(GT)₁TG SEQ ID NO:29 | 4.1x | 10.5x |
| ATGTAT-AT(GT)₁AT SEQ ID N0:30 | 4.8x | 9.8x |
| AGGTGA-AG(G1)₁GA SEQ ID NO:31 | 1.9x | 4.9x |
| GAGTGA-GA(GT)₁GA SEQ ID NO:32 | 1.8x | 5.8x |
| GGGTCT-GG(GT)₁CT SEQ ID NO:33 | 12x | 3.1x |
| CCGTGG-CC(GT),GG SEQ ID NO:34 | 0.0x | 10.8x |
| GGGTCC-GG(GT)₁CC SEQ ID NO:35 | 1.9x | 21.3x |
| CTGTCT-CT(GT)₁CT SEQ ID NO:36 | 2.0x | 15.9x |
| TCGTTC-TC(GT)₁TC SEQ ID NO:37 | 2.2x | 12.9x |
| CGGTGC-CG(GT)₁GC SEQ ID N0:38 | 0.2x | 6.9x |
| TTGTG-TT(GT)₁GG SEQ ID NO:39 | 0.0x | 6.6x |
| GGGTT-GG(GT)₁TT SEQ ID NO:40 | -1.2x | 14.0x |
| GGTTGG-GG(TT)₁GG SEQ ID NO:41 | 3.3x | 16.0x |
| GGAAG-GG(AA)₁G SEQ ID NO:42 | 4.1x | 29.2x |
| GGCCGG-GG(CC)GG SEQ ID NO:43 | 3.1x | 17.1x |
| GGGGGG-GG(GG)₁GG SEQ ID NO:44 | 0.0x | 15.,1x |
| GGGAGG-GG(GA)₁GG SEQ ID NO:45 invention | -1.6x | 23.2x |
| GGGCGG-GG(GC)₁GG SEQ ID NO:46 | 2.3x | 9.8x |
| TTAGGG-TT(AG)₁GG SEQ ID NO:49 | 2.0x | 6.7x |

As shown in Table 13 6 base GT, AG, CG, GG, AGT and CGT sequences increased THP-1 cell production of the cytokines IL-12 and IL-6.

Table 14 summarizes the induction of IL-12 and IL-6 synthesis by 6 base sequences.

**Table 14**

| IL-6 and IL-12 synthesis induced by 6 base sequences | | |
|---|---|---|
| Fold increase | IL-12 synthesis SEQ ID NOs: | IL-6 synthesis SEQ ID NOs: |
| ≤ 2.0 | 9, 31,32, 33, 34, 35, 36, 38, 39,40,44,45.49 | |
| > 2.0 and ≤ 10.0 | 10, 23, 24, 25, 26, 27, 28, 29, 30, 37, 40, 41, 42, 43, 44, 45 | 9, 10, 23, 24, 25, 26, 30, 3 1, 32, 33, 38, 39, 46, 49 |
| > 10.0 | | 25, 27, 29, 34, 35, 36, 37, 40,41,42,43,44,45 |

BCG derived sequences A-3 (SEQ ID NO:73), A-4 (SEQ ID NO:74), A-6 (SEQ ID NO:75), A-7 (SEQ ID NO:76), M3 (SEQ ID NO:77) and Alpha 1 (SEQ ID NO:78) are reported to activate NK cells *in vivo* (Kataoka et al. Jpn. J. Cancer Res. 83:244, 1992). THP-1 human acute monocytic leukemia cells were incubated with 45 base BCG-derived sequences and production of the cytokines IL-12 and IL-6 was determined (Table 15).

**Table 15 (comparison)**

| Cytokine production by THP-1 human acute monocytic leukemia cells | | |
|---|---|---|
| SEQUENCE | **↑**IL-12 | ↑IL-6 |
| BCG A-1 | | |
| AAAGAGGGGCATGACCCGGTGC | 1.9 x | 2.6 x |
| GGGGCTTCTTGCACTCGGCATAG SEQ ID NO:69-(45 bases) | | |
| BCG A-2 | | |
| AAAAGAAGTGGGGTGCCCCCAC | 1.6 x | 3.9 x |
| GATCACCAACGATGGTGTGTCCA SEQ ID NO:70-(45 bases) | | |
| BCG A-3 | | |
| TCCATCGCCAAGGAGATCGAGC | 1.7 x | 2.5 x |
| TGGAGGATCCGTACGAGAAGATC SEQ ID NO:71-(45 bases) | | |
| BCG A-4 | | |
| ACCGATGACGTCGCCGGTGACG | 0.9 x | 1.8 x |
| GCAACACGACGGCCACCGTGCTG SEQ ID NO:72-(45 bases) | | |
| BCG A-6 | | |
| ACGAGACCACCATCGTCGAGGG | 2.1 x | 3.9 x |
| CGCCGGTGACACCGACGCCATCG SEQ ID NO:73-(45 bases) | | |
| BCG A-7 | | |
| GCCGAGAAGGTGCGCAACCTGC | 0.5 x | N.D. |
| CGGCTGGCCACGGACTGAACGCT SEQ ID NO:74-(45 bases) | | |
| BCG M-3 | | |
| ACGCCGACGTCGTCTGTGGTGG | 1.6 x | 2.8 x |
| GGTGTCTACCGCCAACGCGACGG SEQ ID NO:75-(45 bases) | | |
| BCG ALPHA-1 | | |
| CGACTACAACGGCTGGGATATC | 1.1 x | 2.1 x |
| AACACCCCGGCGTTCGAGTGGTA SEQ ID NO:76-(45 bases) | | |

As shown in Table 15, 45 base BCG derived sequences minimally increased THP-1 cell production of IL-12 and IL-6.

### Example 12 (comparison)

### Induction of IL-12 production by phosphodiester and phosphorotothioate sequences

THP-1 human acute monocytic leukemia cells were incubated with 6 base GT sequences, having either an oxygen (phosphodiester) or a sulfur (phosphorothioate) as the nonbridging atom on the phosphate groups and production of the cytokine IL-12 was determined (Table 16).

**Table 16**

| IL-12 production by THP-1 human acute monocytic leukemia cells | | |
|---|---|---|
| SEQUENCE | INCREASE | |
| | PHOSPHODIESTER | PHOSPHOROTHIOAT |
| TGTGTG-(TG)₃ (6 bases) | 1.8x | -0.1x |
| SEQ ID NO:9 phosphodiester; phosphorothioate | | |
| GTGTGT-(GT)₃ (6 bases) | 2.2x | -0.2x |
| SEQ ID NO:10 phosphodiester; phosphorothioat | | |
| TTTGT-TT(TG)₁TT (6 bases) | 3.5x | 0.1x |
| SEQ ID NO:23 phosphodiester, phosphorothioat | | |
| GGTGGG-GG(TG)₁GG (6 bases) | 3.7x | -0.1x |
| SEQ ID NO:24 phosphodiester; phosphorothioat | | |
| GGGTGG-GG(GT)₁GG (6 bases) | 2.0x | 0.0x |
| SEQ ID NO:25 phosphodiester; phosphorothioat | | |
| TTGTTT-TT(GT)₁TT (6 bases) | 3.8x | -0.1x |
| SEQ ID NO:26 phosphodiester; phosphorothioat | | |

As shown in Table 16 substitution of a sulfur atom (phosphorothioate) for a nonbridging oxygen atom (phosphodiester) on the phosphate groups resulted in a significant decrease in THP-1 cell production of IL-12.

THP-1 human acute monocytic leukemia cells were incubated with 6 base GT SEQ ID NO:25, having either an oxygen (phosphodiester) or a sulfur (phosphorothioate) as the nonbridging atom on the phosphate groups and production of the cytokine IL-12 was determined (Table 17).

**Table 17**

| IL-12 production by THP-1 human acute monocytic leukemia cells | |
|---|---|
| SEQUENCE* | INCREASE |
| GₒGₒGₒTₒGₒGₒ-GₒGₒ(GₒTₒ)₁GₒGₒ; (oxygen atom: base 1 to 6) | 2.0 |
| SEQ ID NO:25-(6 bases) | |
| GₒGₒGₛTₒGₒGₒ-GₒGₒ(GₛTₒ)₁GₒGₒ; (oxygen atom: base 1,2,4,5,6; sulfur atom base 3) | 0.1 |
| SEQ ID NO:25-(6 bases) | |
| GₒGₒGₒT₁GₒGₒ-GₒGₒ(GₒTₛ)₁GₒGₒ; (oxygen atom: base 1,2,3,5,6; sulfur atom base 4) | 0.2x |
| SEQ ID NO :25-(6 bases) | |
| GₒGₒGₛTₛGₒGₒ-GₒGₒ(GₛTₛ)₁GₒGₒ; (oxygen atom: base 1,2,5,6; sulfur atom: base 3,4) | 0.5x |
| SEQ ID NO:25-(6 bases) | |
| GₛGₒGₒTₒGₒGₛ-GₛGₒ(GₒTₒ)₁GₒGₛ; (oxygen atom: base 2,3,4,5; sulfur atom: base 1,6) | -0.1x |
| SEQ ID NO:25-(6 bases) | |
| GₒGₛGₒTₒGₛGₒ- GₒGₛ(GₒTₒ)₁GₛGₒ; (oxygen atom: position 1,3,4,6; sulfur atom: position 2,5) | -0.1x |
| SEQ ID NO:25-(6 bases) | |
| GₛGₛGₒTₒGₛGₛ -GₛGₛ(GₒTₒ)₁GₛGₛ; (oxygen atom: position 3,4; sulfur atom: | 0 |
| position 1,2,5,6) | |
| SEQ ID NO:25-(6 bases) | |
| GₛGₛGₛTₛGₛGₛ- GₛGₛ(GₛTₛ)₁GₛGₛ; (sulfur atom: position 1 to 6) | 0 |
| SEQ ID NO:25(6 bases) | |

| | |
|---|---|
| *Note: "o" represents an oxygen atom and "s" represents a sulfur atom on the phosphate group | |

As shown in Table 17 substitution of a sulfur atom (phosphorothioate) for a nonbridging oxygen atom (phosphodiester) in 6 base GT SEQ ID NO:25 resulted in a significant decrease in THP-1 cell production of IL-12.

### Example 3

### Stimulation of cytokine synthesis in human peripheral blood mononuclear cells

Peripheral blood mononuclear cells (hereinafter, "PBMCs") were isolated from 7 healthy humans by Ficoll-Hypaque (Amersham Pharmacia Biotech, Baie d'Urfée, Québec, Canada) by density gradient centrifugation of whole blood. PBMCs were incubated with 6 base GT, AGT, CGT, AG, CG and GG sequences and production of the cytokines IL- beta, IL-6, IL-10 and IL-12 were determined.

**Table 18**

| Cytokine production by human PBMC | | | | |
|---|---|---|---|---|
| SEQUENCES | IL-1beta fold increase: mean +/- SD (range) | IL-6 fold increase: mean +/- SD (range) | IL-10 fold increase: mean +/- SD (range) | IL-12 fold increase: mean +/- SD (range) |
| TG(TG)₁TG | 1.2 +/- 0.4 | 8.3 +/- 12.8 | 1.0 +/- 0.1 | 2.7 +/- 2.6 |
| SEQ ID NO:9-(6 bases) | (0.8-1.5) | (1.2-37.0) | (0.9-1.1) | (1.0-6.6) |
| GT(GT)₁GT | 2.0 +/- 1.6 | 9.8 +/ 14.2 | 1.0 +/- 0.1 | 4.0 +/- 6.3 |
| SEQ ID NO:10-(6 bases) | (0.9-3.8) | (0.8-39.1) | (0.9-1.1) | (0.9-18.1) |
| TG(TG)₄TG | 2.4 +/- 1.9 | 12.1 +/- 6.5 | 1.2 +/- 0.4 | 4.4 +/- 53 |
| SEQ ID NO:13-(12 bases) | (0.9-4.5) | (2.9-20.8) | (0.9-1.9) | (1.2-15.0) |
| GT(GT)₄GT | 1.1 +/- 0.2 | 2.0 +/- 1.5 | 1.0 +/- 0.1 | 2.0 +/- 1.1 |
| SEQ 1D NO:14 -(12 bases) | (0.9-1.3) | (0.9-4.9) | (0.9-1.2) | (0.9-2.6) |
| TT(TG)₁TT | 1.0 +/- 0.1 | 11.8 +/- 9.0 | 1.0 +/ 0.1 | 1.1 +/- 0.3 |
| SEQ ID NO:23 -(6 bases) | (0.9-1.0) | (1.3-25.6) | (0.9-1.1) | (0.9-1.6) |
| GG(TG)₁GG | 0.9 +/- 0.1 | 15.9 +/- 14.9 | 2.4 +/- 2.9 | 2.3 +/- 1.6 |
| SEQ ID NO. 24 (6 bases) | (0.9-1.0) | (0.7-37.1) | (1.0-7.5) | (0.9-5.5) |
| GG(GT)₁GG | 1.0 +/- 0.1 | 20.9 +/- 18.0 | 11.6 +/- 1.2 | 132 +/- 11.5 |
| SEQ ID NO:25 -(6 bases) | (1.0-1.2) | (1.6-50.0) | (9.9-132) | (1.0-26.8) |
| TT(GT)₁TT | 1.5 +/- 0.9 | 5.8 +/- 8.0 | 1.0 +/- 0.1 | 1.6 +/- 1.3 |
| SEQ ID NO:26-(6 bases) | (0.9-2.5) | (0.5-21.7) | (1.0-1.2) | (0.8-4.5) |
| AA(GT)₁AA | 1.3 +/- 0.5 | 9.6 +/- 7.3 | 1.0 +/- 0.1 | 2.4 +/- 22 |
| SEQ ID NO: 27-(6 bases) | (0.9-1.8) | (1.8-16.0) | (0.9-1.1) | (0.8-6.7) |
| CC(GT)₁CC | 2.1 +/- 1.8 | 10.4 +/- 13.0 | 1.0 +/- 0.1 | 5.9 +/- 10.7 |
| SEQ ID NO: 28-(6 bases) | (0.8-4.1) | (1.4-35.8) | (1.0-1.1) | (0.9-30.0) |
| TG(GT)₁TG | 1.7 +/- 1.0 | 9.3 +/- 12.1 | 1.0 +/- 0.1 | 3.3 +/- 4.2 |
| SEQ ID NO: 29-(6 bases) | (1.1-2.8) | (0.8-33.8) | (1.0-1.1) | (0.8-12.7) |
| AT(GT)₁AT | 1.1 +/- 0.2 | 4.6 +/- 4.4 | 1.0 +/- 0.1 | 1.3 +/- 0.2 |
| SEQ ID NO: 30-(6 bases) | (1.0-1.4) | (1.6-14.4) | (0.9-1.1) | (1.0-1.6) |
| CT(GT)₁CT | 1.2 +/- 0.3 | 5.3 +/- 3.6 | 1.0 +/- 0.1 | 1.2 +/- 0.3 |
| SEQ ID NO: 36-(6 bases) | (1.0-1.5) | (0.9-10.6) | (0.9-1.2) | (0.9-1.8) |
| TC(GT)₁TC | 12+/-0.4 | 7.5+/-9.8 | 1.0+/-0.1 | 1.4+/-1.1 |
| SEQ ID NO:37-(6 bases) | (0.9-1.6) | (0.7-27.0) | (1.0-1.1) | (0.9-3.8) |
| GG(TT)₁GG | 3.2+/-3.0 | 7.8+/-12.8 | 1.0+/-0.1 | 4.9+/-8.6 |
| SEQ ID NO:41-(6 bases) | (0.8-6.5) | (0.9-36.4) | (0.9-1.1) | (0.8-243) |
| GG(AA)₁GG | 3.5 +/- 3.9 | 11.9+/-5.8 | 1.1 +/ 02 | 3.0+/-2.7 |
| SEQ 1D NO:42-(6 bases) | (0.8-8.0) | (12-15.6) | (0.9-1.5) | . (1.1-8.6) |
| GG(CC)₁GG | 1.5 +/- 0.9 | 14.9 +/-102 | 1.2 +/- 03 | 2.5 +/- 2.0 |
| SEQ ID NO:43-(6 bases) | (0.8-2.5) | (12-29.8) | (1.0-1.8) | (1.0-7.0) |
| GG(GG)₁GG | 7.1+/- 9.4 | 21.0 +/- 14.7 | 1.7+/-1.6 | 7.0+/-12.5 |
| SEQ ID NO:44-(6 bases) | (0.8-17.9) | (4.6-42.0) | (0.9-4.6) | (1.3-35.3) |
| GG(GA)₁GG (invention) | 13.6 +/- 14.9 | 24.7 +/- 16.5 | 6.0+/-5.5 | 20.7 +/- 10.3 |
| SEQ ID NO:45-(6 bases) | (1.2-30.2) | (5.9-50.0) | (2.1-15.5) | (5.7-35.3) |
| GG(GC)₁GG | 1.2+/-0.3 | 15.8 +/- 16.2 | 1.0 +/- 0.1 | 3.0 +/- 3.8 |
| SEQ ID NO:46-(6 bases) | (1.0-1.5) | (1.3-37.8) | (0.9-1.1) | (1.1-10.7) |

As shown in Table 18 6 base GT, AGT, CGT, AG, CG and GG sequences increased human PBMC cell production of the cytokines IL-1beta, IL-6, IL-10 and IL-12.

### Example 14

### Cytokine synthesis by chimpanzee peripheral blood mononuclear cells

PBMCs were isolated from 4 healthy chimpanzees as in Example 35. Chimpanzee PBMCs were incubated with 6 base GT, AGT, CGT, AG, CG and GG sequences and production of the cytokines IL-10, IL-12 and TNF-alpha was determined (Table 19).

**Table 19**

| Cytokine production by chimpanzees PBMC | | | |
|---|---|---|---|
| SEQUENCES | IL-10 fold increase: mean +/- SD (range) | IL-12 fold increase: mean +/- SD (range) | TNF-alpha fold increase: mean +/- SD (range) |
| TG(TG)₁TG | 2.3+/-1.3 | 13.5+/-8.9 | 11.3+/-6.9 |
| SEQ ID NO:9-(6 bases) | (1.3-4.1) | (2.8-21.1) | (53-19.5) |
| GT(GT)₁GT | 4.0+/-2.1 | 14.0+/-8.5 | 12.9+/-6.4 |
| SEQ ID NO:1-(6 bases) | (1.8-6.7) | (3.0-21.3) | (6.5-19.6) |
| TT(TG)₁TT | 1.5+/-0.6 | 12.9+/-82 | 9.0+/-5.5 |
| SEQ ID NO:23-(6 bases) | (1.0-2.4) | (2.7-20.1) | (4.1-14.4) |
| GG(TG)₁GG | 2.9+/-1.5 | 143 +/- 9.1 | 11.9 +/- 7.0 |
| SEQ ID NO:24-(6 bases) | (1.3-4.8) | (3.0-22.5) | (5.8-19.8) |
| GG(GT)₁GG | 2.5 +/- 1.5 | 13.5+/-8.4 | 11.7 +/-; 6.7 |
| SEQ ID NO:25-(6 bases) | (1.4-4.6) | (2.8-20.8) | (5.9-19.6) |
| TT(GT)₁TT | 1.4+/-0.9 | 12-3+/-8.5 | 7.5+/-4.6 |
| SEQ ID NO:26-(6 bases) | (1.1-2.1) | (2.5-20.1) | (3.4-13.1) |
| AA(GT)₁AA | 2.1+/-1.1 | 132 +/- 82 | 7.9 +/- 3.9 |
| SEQ ID NO:27(6 bases) | (1.1-3.7) | (2.8-19.8) | (4.6-12.0) |
| CC(GT)₁CC | 3.8+/-2.8 | 13.3+/-8.4 | 10-3+/-5.7 |
| SEQ ID NO:28-(6 bases) | (1.5-7.7) | (2.8-20.4) | (5.0-15.8) |
| TG(GT)₁TG | 3.1 +/-2.0 | 13.6+/-8.8 | 12.4 +/-7.3 |
| SEQ ID NO:29-(6 bases) | (1.6-5.9) | (2.9-21.9) | (6.1-20.8) |
| AT(GT)₁AT | 1.4 +/-0.4 | 10.7 +/-7.0 | 5.9 +/-3.3 |
| SEQ ID NO:30-(6 bases) | (1.2-1.9) | (2.5-18.6) | (3.4-10.5) |
| CT(GT)₁CT | 3.0+/-2.1 | 13.4 +/- 8.9 | 12.4 +/- 6.3 |
| SEQ ID NO:36-(6 bases) | (1.2-5.9) | (2.8-20.4) | (7.0-19.6) |
| TC(GT)₁TC | 3.4+/-2.6 | 14.1+/-10.0 | 11.4+/-6.4 |
| SEQ ID NO:37-(6 bases) | (1.4-7.1) | (2.4-24.9) | (6.1-19.3) |
| GG(TT)₁GG | 9.1+/-7.7 | 15.3+/-10.0 | 14.1+/-7.3 |
| SEQ ID NO:41(6 bases) | (3.0-20.3) | (2.9-25.9) | (7.7+/- 23.2) |
| GG(AA)₁GG | 9.9+/-8.9 | 15.6 +/- 10.6 | 14.4 +/-7.1 |
| SEQ ID NO:42-(6 bases) | (2.6-22.7) | (2.6-26.6) | (8.0-22.9) |
| GG(CC)₁GG | 13.6+/-9.0 | 15.1+/-10.2 | 14.0+/-6.6 |
| SEQ ID NO:43-(6 bases) | (4.3-26.7) | (2.8-26.1) | (7.9-22.3) |
| GG(GG)₁GG | 11.2+/-9.1 | 15.1+/-10.0 | . 13.8+/-6.5 |
| SEQ ID NO:44-(6 bases) | (3.9-24.3) | (2.9-25.9) | (7.6-21.8) |
| GG(GA)₁GG (invention) | 9.9+/-9.2 | 15.9 +/-10.7 | 14.5+/-6.9 |
| SEQ ID NO:45-(6 bases) | (2.6-23.1) | (2.9-26.9) | (7.9-23.0) |
| GG(GC)₁GG | 4.7+/-3.4 | 15.8+/-10.4 | 14.1+/-6.6 |
| SEQ ID NO:46-(6 bases) | (1.7-9.3) | (3.0-26.2) | (8.3-21.7) |

As shown in Table 19 6 base GT, AGT, CGT, AG, CG and GG sequences increased chimpanzee PBMC cell production of the cytokines IL-10 and IL-12 and TNF-alpha.

### Example 15

### Cytokine synthesis by rhesus monkey peripheral blood mononuclear cells

PBMCs were isolated from 4 healthy rhesus monkeys as in Example 35. PBMCs were incubated with 6 base GT, AGT, CGT, AG, CG ands GG sequences and production of the cytokines IL-6, IL-12 and TNF-alpha was determined (Table 42).

**Table 20**

| Cytokine production by rhesus monkeys PBMC | | | |
|---|---|---|---|
| SEQUENCES | IL-10 fold increase: mean +/- SD (range) | IL-12 fold increase: mean +/- SD (range) | TNF-alpha fold increase: mean +/- SD (range) |
| TG(TG)₁TG | 1.1+/-02 | 10.6+/-4.2 | 14.3+/-6.3 |
| SEQ ID NO:9-(6 bases) | (0.9-1.3) | (5.6-143) | (6.2-21.2) |
| GT(GT)₁GT | 1.3+/-0.1 | 10.7+/-4.1 | 16.1+/-42 |
| SEQ ID NO:10-(6 bases) | (1.1-1.4) | (6.1-15.8) | (11.0-21.6) |
| TT(TG)₁TT | 1.0+/-0.1 | 6.7+/-3.6 | 4.4 +/- 3.8 |
| SEQ ID NO:23-(6 bases) | (0.8-1.0) | (3.6-11.7) | (1.3-9.5) |
| GG(TG)₁GG | 1.0 +/- 0.1 | 11-2+/-4.9 | 14.5 +/- 5.4 |
| SEQ ID N0:24-(6 bases) | (1.0-1.1) | (5.6-16.8) | (7.7-20.0) |
| GG(GT)₁GG | 1.0 +/- 0.1 | 10.6 +/- 4.7 | 12.5 +/- 5.2 |
| SEQ ID NO:25-(6 bases) | (1.0-1.1) | (5.5-16.5) | (6.2-18.6) |
| TT(GT)₁TT | 1.0 +/- 0.1 | 4.9+/-2.9 | 2.1 +/- 1.0 |
| SEQ ID NO:26-(6 bases) | (1.0-12) | (2.6-8.8) | (1.3-3.4) |
| AA(GT)₁AA | 0.9 +/- 0.1 | 7.6 +/- 2.6 | 6.0+/-5.0 |
| SEQ ID NO:27-(6 bases) | (0.9-1.0) | (5.9-11.5) | (2.4-13.4) |
| CC(OT)₁CC | 1.1+/-0.2 | 10.1 +/- 3.7 | 14.2+/-3.7 |
| SEQ ID NO:28-(6 bases) | (0.9-1.2) | (6.2-14.2) | (9.6-18.6) |
| TG(GT)₁TG | 1.1+/-02 | 11.1+/-4.2 | 16.5+/-2.7 |
| SEQ ID NO:29-(6 bases) | (0.9-1.2) | (6.5-15.7) | (14.0-19.1) |
| AT(GT)₁AT | 1.9+/-1.4 | 6.0+/-1.9 | 6.9+/-4.7 |
| SEQ ID NO:30-(6 bases) | (1.0-4.0) | (5.6-8.5) | (2.2-13.4) |
| GT(GT)₁CT | 1.0+/-0.1 | 10.7+/-4.6 | 153+/-3.6 |
| SEQ ID NO:36-(6 bases) | (0.9-1.1) | (6.2-16.4) | (11.2-19.8) |
| TC(GT)₁TC | 1.1+/-02 | 10.0+/-3.8 | 14.7+/-3.1 |
| SEQ ID NO:37-(6 bases) | (0.9-1.3) | (6.3-14.1) | (13.7-19.1) |
| GG(TT)₁GG | 1.9+/-1.5 | 11.5+/-5.9 | 14.1+/-8.2 |
| SEQ ID NO:41 -(6 bases) | (1.0-4.1) | (4-3-17-2) | (2.5-20.7) |
| GG(AA)₁GG | 1.2 +/- 0.2 | 11.9 +/- 5.4 | 16.5 +/- 4.5 |
| SEQ ID NO:42 -(6 bases) | (1.0-1.4) | (5.9-17.1) | (11.4-21.2) |
| GG(CC)₁GG | 1.0 +/- 0.2 | 11.7 +/- 4.8 | 16.9 +/- 3.5 |
| SEQ ID NO:43-(6 bases) | (0.8-1.2) | (6.2-16.4) | (13.9-21.1) |
| GG(GG)₁GG | 2.1 +/- 1.0 | 10.7 +/- 5.6 | 13.9 +/- 8.5 |
| SEQ ID NO:44-(6 bases) | (1.1-3.5) | (3.7-15.9) | (2.0-20.9) |
| GG(GA)₁GG (invention) | 1.1 +/- 0.1 | 11.9 +/- 4.5 | 16.7 +/- 4.6 |
| SEQ ID NO:45 -(6 bases) | (1.0-1.3) | (6.8-16.0) | (11.6-21.5) |
| GG(GC)₁GG | 1.2 +/- 0.2 | 11.0 +/- 4.4 | 16.8 +/- 4.1 |
| SEQ ID NO:46-(6 bases) | (1.0-1.4) | (6.3-16.1) | (11.9-21.8) |

As shown in Table 20, 6 base GT, AGT, CGT, AG, CG and GG sequences increased rhesus monkey PBMC cell production of the cytokines IL-10, IL-12 and TNF-alpha.

### Example, 16 (comparison)

### Effect of 6 base GT SEQ ID NO:25 of 6 base GT SEQ ID NO:25 + 5-fluorouracil and of 6 base GT SEQ ID NO:25 + tamoxifen on MCF-7 human breast tumors

MCF-7 human breast cancer cells are implanted subcutaneously as xenografts, in female nude BALB/c mice. The mice are divided into 6 groups of 10 mice. On day 0, group 1 mice receive saline, group 2 mice receive 6 base GT SEQ ID NO:25, group 3 mice receive receive 5-fluorouracil, group 4 mice receive tamoxifen, group 5 mice receive 6 base GT SEQ ID NO:25 + 5-fluorouracil and group 6 mice receive 6 base GT SEQ ID NO:25 + tamoxifen. After 4 weeks of treatment, the mice are sacrificed and tumor mass is determined. Group 1 mice have the most tumor mass, groups 2, 3 and 4 mice have less tumor mass than group 1 mice and groups 5 and 6 mice have less tumor mass than groups 1, 2,.3 and 4 mice.

### Example 17

### Effect of 3 and 6 base GT sequences and 45 base BCG A-3 sequence on LNCaP human prostate cancer tumors

LNCaP human prostate cancer cells are implanted subcutaneously, as xenografts, in male nude nu/nu mice. The mice are divided into 5 groups of 10 mice. On day 0, group 1 mice receive saline, group 2 mice receive 3 base SEQ ID NO:8, group 3 mice receive 6 base GT SEQ ID NO:25, group 4 mice receive 6 base AG SEQ ID N0:45 and group 5 mice receive 45 base BCG A-3 SEQ ID N0:69. After 4 weeks of treatment, the mice are sacrificed and tumor mass is determined. Group 1 mice have the most tumor mass, group 5 mice have less tumor mass than group 1 mice and groups 2, 3 and 4 mice have less tumor mass than groups 1 and 5 mice.

### Example 18

### Effect of 3, 6, 8 and 27 base sequences on EL-4 murine T lymphomas

EL-4 murine T lymphoma cells are implanted into C57/BL6 mice. The mice are divided into 6 groups of 10 mice. On day 0, group 1 mice receive saline, group 2 mice receive 3 base GT SEQ ID NO:8, group 3 mice receive 6 base SEQ ID NO:25, group 4 mice receive 6 base AG SEQ ID NO:45, group 5 mice receive 18 base GT SEQ ID NO:18 and group 6 mice receive 27 base GT SEQ ID NO:1. After 4 weeks of treatment, the mice are sacrificed and tumor mass is determined. Group 1 mice have the most tumor mass, groups 2, 3, 4, 5 and 6 mice have less tumor mass than group 1 mice.

### Example 19

Human colon cancer cell lines are maintained as adherent cell cultures. Cells in the exponential growth phase are treated with 2-20 base GT, GA, GC or GG sequences in the dose range 0 µg/ml to 100 µl/ml for 24-72 hours. Inhibition of cell proliferation is measured by MTT reduction, cell cycle arrest by flow cytometry and apoptosis by annexin-V binding or NuMA release. GT, GA, GC or GG sequences inhibit proliferation, induce cell cycle arrest and induce apoptosis in the colon cancer cell lines.

SCID mice bearing subcutaneous human colorectal cancer cell lines are treated with saline or with 2-20 base GT, GA, GC or GG sequences, having anti-cancer activity against human colorectal cancer cell lines *in vitro.* Mice treated with 2-20 base GT, GA, GC or GG sequences, having anti-cancer activity against human colorectal cancer cell lines *in vitro,* show a significant reduction in tumor mass compared with mice treated with saline.

## Claims

1. A composition comprising an isolated 3'-OH, 5'-OH synthetic phosphodiester nucleotide sequence of SEQ ID NO:45:gggagg and further comprising a pharmaceutically acceptable carrier.

2. The composition of claim 1, further comprising a chemotherapeutic agent.

3. The composition of claims 1 or 2, wherein the chemotherapeutic agent is selected from the group consisting of an antimetabolite, an alkylating agent and a hormone antagonist.

4. Use of the composition of any of claims 1 to 3 in the preparation of a medicament for inducing a response selected from the group consisting of induction of cell cycle arrest, inhibition of proliferation, activation of caspases and induction of apoptosis in cancer cells, and production of cytokines by immune system cells, wherein the cytokines are selected from the group consisting of IL-1-beta, IL-6, IL-10, IL-12, and TNF-alpha.

5. Use of the composition of any of claims 1 to 3 in the preparation of a medicament for treating cancer.

6. The use of any of claims 4 to 5, wherein the cancer is selected from the group consisting of a primary carcinoma, a secondary carcinoma, a primary sarcoma and a secondary sarcoma.

7. The use of claim 6, wherein the cancer is selected from the group consisting of leukemia, lymphoma, breast, prostate, colorectal, ovarian and bone cancer.

## Patentansprüche

1. Zusammensetzung umfassend eine isolierte 3'-OH, 5'-OH synthetische Phosphodiesternukleotidsequenz gemäß SEQ ID NO:45:gggagg und ferner umfassend einen pharmazeutisch annehmbaren Träger.

2. Zusammensetzung nach Anspruch 1, ferner umfassend ein chemotherapeutisches Mittel.

3. Zusammensetzung nach Anspruch 1 oder 2, wobei das chemotherapeutische Mittel ausgewählt ist aus der Gruppe bestehend aus einem Antimetaboliten, einem alkylierenden Mittel und einem Hormonantagonisten.

4. Verwendung der Zusammensetzung nach einem der Ansprüche 1-3 bei der Herstellung eines Medikaments zur Induzierung einer Reaktion ausgewählt aus der Gruppe bestehend aus Induktion von Zellzyklusarrest, Hemmung von Proliferation, Aktivierung von Kaspasen und Induktion von Apoptose in Krebszellen sowie Produktion von Zytokinen durch Immunsystemzellen, wobei die Zytokine ausgewählt sind aus der Gruppe bestehend aus IL-1-Beta, IL-6, IL-10, IL-12 und TNF-Alpha.

5. Verwendung der Zusammensetzung nach einem der Ansprüche 1 bis 3 bei der Herstellung eines Medikaments zur Behandlung von Krebs.

6. Verwendung nach einem der Ansprüche 4 oder 5, wobei der Krebs ausgewählt ist aus der Gruppe bestehend aus einem Primärkarzinom, einem Sekundärkarzinom, einem Primärsarkom und einem Sekundärsarkom.

7. Verwendung nach Anspruch 6, wobei der Krebs ausgewählt ist aus der Gruppe bestehend aus Leukämie, Lymphomakrebs, Brustkrebs, Prostatakrebs, kolorektalem Krebs, ovarialem Krebs und Knochenkrebs.

## Revendications

1. Composition comprenant une séquence de nucléotides phosphodiester synthétiques 3'-OH, 5'-OH de SEQ ID NO:45 :gggagg et comprenant en outre un véhicule acceptable en pharmacie.

2. Composition selon la revendication 1, comprenant en outre un agent chimiothérapique.

3. Composition selon les revendications 1 ou 2, dans laquelle l'agent chimiothérapique est choisi dans le groupe constitué par un antimétabolite, un agent d'alkylation et un antagoniste hormonal.

4. Utilisation de la composition selon l'une quelconque des revendications 1 à 3, dans la préparation d'un médicament pour induire une réponse choisie dans le groupe constitué par l'induction de l'arrêt du cycle cellulaire, l'inhibition de la prolifération, l'activation des caspases et l'induction de l'apoptose dans les cellules cancéreuses, et la production de cytokines par les cellules du système immunitaire, dans laquelle les cytokines sont choisies dans le groupe constitué par l'IL-1-bêta, l'IL-6, l'IL-10, l'IL-12 et le TNF-alpha.

5. Utilisation de la composition selon l'une quelconque des revendications 1 à 3, dans la préparation d'un médicament pour traiter le cancer.

6. Utilisation selon l'une quelconque des revendications 4 à 5, dans laquelle le cancer est choisi dans le groupe constitué par un carcinome primaire, un carcinome secondaire, un sarcome primaire et un sarcome secondaire.

7. Utilisation selon la revendication 6, dans laquelle le cancer est choisi dans le groupe constitué par la leucémie, le lymphome, le cancer du sein, le cancer de la prostate, le cancer colorectal, le cancer de l'ovaire et le cancer des os.


### SEQUENCE LISTING

<110> Phillip, Nigel
   Filion, Mario
<120> Therapeutically Useful Synthetic Oligonucleotides
<130> 6857-21
<150> 60/170,325
   <151> 1999-12-13
<150> 60/228,925
   <151> 2000-08-29
<160> 87
<170> PatentIn version 3.0
<210> 1
   <211> 27
   <212> DNA
   <213> Synthetic Oligonucleotide
<400> 1
   gtgtgtgtgt gtgtgtgtgt gtgtgtg 27
<210> 2
   <211> 27
   <212> DNA
   <213> Synthetic Oligonucleotide
<400> 2
   gggtgggtgg gtgggtgggt gggtggg 27
<210> 3
   <211> 27
   <212> DNA
   <213> Synthetic Oligonucleotide
<400> 3
   gggggtgggg gtgggggtgg gggtggg 27
<210> 4
   <211> 27
   <212> DNA
   <213> Synthetic Oligonucleotide
<400> 4
   gggggggtgg gggggtgggg gggtggg 27
<210> 5
   <211> 27
   <212> DNA
   <213> Synthetic Oligonucleotide
<400> 5
   tgtgtgtgtg tgtgtgtgtg tgtgtgt 27
<210> 6
   <211> 27
   <212> DNA
   <213> Synthetic Oligonucleotide
<400> 6
   tctctctctc tctctctctc tctctct 27
<210> 7
   <211> 3
   <212> DNA
   <213> Synthetic Oligonucleotide
<400> 7
   tgt 3
<210> 8
   <211> 3
   <212> DNA
   <213> Synthetic Oligonucleotide
<400> 8
   gtg 3
<210> 9
   <211> 6
   <212> DNA
   <213> Synthetic Oligonucleotide
<400> 9
   tgtgtg 6
<210> 10
   <211> 6
   <212> DNA
   <213> Synthetic Oligonucleotide
<400> 10
   gtgtgt 6
<210> 11
   <211> 9
   <212> DNA
   <213> Synthetic Oligonucleotide
<400> 11
   tgtgtgtgt 9
<210> 12
   <211> 9
   <212> DNA
   <213> Synthetic Oligonucleotide
<400> 12
   gtgtgtgtg 9
<210> 13
   <211> 12
   <212> DNA
   <213> Synthetic Oligonucleotide
<400> 13
   tgtgtgtgtg tg 12
<210> 14
   <211> 12
   <212> DNA
   <213> Synthetic Oligonucleotide
<400> 14
   gtgtgtgtgt gt 12
<210> 15
   <211> 14
   <212> DNA
   <213> Synthetic Oligonucleotide
<400> 15
   tgtgtgtgtg tgtg 14
<210> 16
   <211> 15
   <212> DNA
   <213> Synthetic Oligonucleotide
<400> 16
   gtgtgtgtgt gtgtg 15
<210> 17
   <211> 18
   <212> DNA
   <213> Synthetic Oligonucleotide
<400> 17
   tgtgtgtgtg tgtgtgtg 18
<210> 18
   <211> 18
   <212> DNA
   <213> Synthetic Oligonucleotide
<400> 18
   gtgtgtgtgt gtgtgtgt 18
<210> 19
   <211> 21
   <212> DNA
   <213> Synthetic Oligonucleotide
<400> 19
   tgtgtgtgtg tgtgtgtgtg t 21
<210> 20
   <211> 21
   <212> DNA
   <213> Synthetic Oligonucleotide
<400> 20
   gtgtgtgtgt gtgtgtgtgt g 21
<210> 21
   <211> 24
   <212> DNA
   <213> Synthetic Oligonucleotide
<400> 21
   tgtgtgtgtg tgtgtgtgtg tgtg 24
<210> 22
   <211> 24
   <212> DNA
   <213> Synthetic Oligonucleotide
<400> 22
   gtgtgtgtgt gtgtgtgtgt gtgt 24
<210> 23
   <211> 6
   <212> DNA
   <213> Synthetic Oligonucleotide
<400> 23
   tttgtt 6
<210> 24
   <211> 6
   <212> DNA
   <213> Synthetic Oligonucleotide
<400> 24
   ggtggg 6
<210> 25
   <211> 6
   <212> DNA
   <213> Synthetic Oligonucleotide
<400> 25
   gggtgg 6
<210> 26
   <211> 6
   <212> DNA
   <213> Synthetic Oligonucleotide
<400> 26
   ttgttt 6
<210> 27
   <211> 6
   <212> DNA
   <213> Synthetic Oligonucleotide
<400> 27
   aagtaa 6
<210> 28
   <211> 6
   <212> DNA
   <213> Synthetic Oligonucleotide
<400> 28
   ccgtcc 6
<210> 29
   <211> 6
   <212> DNA
   <213> Synthetic Oligonucleotide
<400> 29
   tggttg 6
<210> 30
   <211> 6
   <212> DNA
   <213> Synthetic Oligonucleotide
<400> 30
   atgtat 6
<210> 31
   <211> 6
   <212> DNA
   <213> Synthetic Oligonucleotide
<400> 31
   aggtga 6
<210> 32
   <211> 6
   <212> DNA
   <213> Synthetic Oligonucleotide
<400> 32
   gagtga 6
<210> 33
   <211> 6
   <212> DNA
   <213> Synthetic Oligonucleotide
<400> 33
   gggtct 6
<210> 34
   <211> 6
   <212> DNA
   <213> Synthetic Oligonucleotide
<400> 34
   ccgtgg 6
<210> 35
   <211> 6
   <212> DNA
   <213> Synthetic Oligonucleotide
<400> 35
   gggtcc 6
<210> 36
   <211> 6
   <212> DNA
   <213> Synthetic Oligonucleotide
<400> 36
   ctgtct 6
<210> 37
   <211> 6
   <212> DNA
   <213> Synthetic Oligonucleotide
<400> 37
   tcgttc 6
<210> 38
   <211> 6
   <212> DNA
   <213> Synthetic Oligonucleotide
<400> 38
   cggtgc 6
<210> 39
   <211> 6
   <212> DNA
   <213> Synthetic Oligonucleotide
<400> 39
   ttgtgg 6
<210> 40
   <211> 6
   <212> DNA
   <213> Synthetic Oligonucleotide
<400> 40
   gggttt 6
<210> 41
   <211> 6
   <212> DNA
   <213> Synthetic Oligonucleotide
<400> 41
   ggttgg 6
<210> 42
   <211> 6
   <212> DNA
   <213> Synthetic Oligonucleotide
<400> 42
   ggaagg 6
<210> 43
   <211> 6
   <212> DNA
   <213> Synthetic Oligonucleotide
<400> 43
   ggccgg 6
<210> 44
   <211> 6
   <212> DNA
   <213> Synthetic Oligonucleotide
<400> 44
   gggggg 6
<210> 45
   <211> 6
   <212> DNA
   <213> Synthetic Oligonucleotide
<400> 45
   gggagg 6
<210> 46
   <211> 6
   <212> DNA
   <213> Synthetic Oligonucleotide
<400> 46
   gggcgg 6
<210> 47
   <211> 6
   <212> DNA
   <213> Synthetic Oligonucleotide
<400> 47
   ggaggg 6
<210> 48
   <211> 6
   <212> DNA
   <213> Synthetic Oligonucleotide
<400> 48
   gtgggg 6
<210> 49
   <211> 6
   <212> DNA
   <213> Synthetic Oligonucleotide
<400> 49
   ttaggg 6
<210> 50
   <211> 2
   <212> DNA
   <213> Synthetic Oligonucleotide
<400> 50
   gt 2
<210> 51
   <211> 2
   <212> DNA
   <213> Synthetic Oligonucleotide
<400> 51
   tg 2
<210> 52
   <211> 4
   <212> DNA
   <213> Synthetic Oligonucleotide
<400> 52
   gtgg 4
<210> 53
   <211> 4
   <212> DNA
   <213> Synthetic Oligonucleotide
<400> 53
   ttgt 4
<210> 54
   <211> 4
   <212> DNA
   <213> Synthetic Oligonucleotide
<400> 54
   gtgt 4
<210> 55
   <211> 4
   <212> DNA
   <213> Synthetic Oligonucleotide
<400> 55
   ttgg 4
<210> 56
   <211> 4
   <212> DNA
   <213> Synthetic Oligonucleotide
<400> 56
   ggtg 4
<210> 57
   <211> 4
   <212> DNA
   <213> Synthetic Oligonucleotide
<400> 57
   tgtt 4
<210> 58
   <211> 4
   <212> DNA
   <213> Synthetic Oligonucleotide
<400> 58
   ggtt 4
<210> 59
   <211> 4
   <212> DNA
   <213> Synthetic Oligonucleotide
<400> 59
   tgtg 4
<210> 60
   <211> 5
   <212> DNA
   <213> Synthetic Oligonucleotide
<400> 60
   ggtgg 5
<210> 61
   <211> 5
   <212> DNA
   <213> Synthetic Oligonucleotide
<400> 61
   gggtg 5
<210> 62
   <211> 7
   <212> DNA
   <213> Synthetic Oligonucleotide
<400> 62
   ggggtgg 7
<210> 63
   <211> 7
   <212> DNA
   <213> Synthetic Oligonucleotide
<400> 63
   gggtggg 7
<210> 64
   <211> 7
   <212> DNA
   <213> Synthetic Oligonucleotide
<400> 64
   tgggtgg 7
<210> 65
   <211> 7
   <212> DNA
   <213> Synthetic Oligonucleotide
<400> 65
   gggtggt 7
<210> 66
   <211> 27
   <212> DNA
   <213> Synthetic Oligonucleotide
<400> 66
   gtgtgtgtgt gtgtgtgtgt gtgtgtg 27
<210> 67
   <211> 27
   <212> DNA
   <213> Synthetic Oligonucleotide
<400> 67
   gggtgggtgg gtgggtgggt gggtggg 27
<210> 68
   <211> 27
   <212> DNA
   <213> Synthetic Oligonucleotide
<400> 68
   gggggtgggg gtgggggtgg gggtggg 27
<210> 69
   <211> 27
   <212> DNA
   <213> Synthetic Oligonucleotide
<400> 69
   gggggggtgg gggggtgggg gggtggg 27
<210> 70
   <211> 6
   <212> DNA
   <213> Synthetic Oligonucleotide
<400> 70
   tgtgtg 6
<210> 71
   <211> 6
   <212> DNA
   <213> Synthetic Oligonucleotide
<400> 71
   gtgtgt 6
<210> 72
   <211> 6
   <212> DNA
   <213> Synthetic Oligonucleotide
<400> 72
   tttgtt 6
<210> 73
   <211> 6
   <212> DNA
   <213> Synthetic Oligonucleotide
<400> 73
   ggtggg 6
<210> 74
   <211> 6
   <212> DNA
   <213> Synthetic Oligonucleotide
<400> 74
   gggtgg 6
<210> 75
   <211> 6
   <212> DNA
   <213> Synthetic Oligonucleotide
<400> 75
   ttgttt 6
<210> 76
   <211> 45
   <212> DNA
   <213> Synthetic Oligonucleotide
<400> 76
   gccgagaagg tgcgcaacct gccggctggc cacggactga acgct 45
<210> 77
   <211> 45
   <212> DNA
   <213> Synthetic Oligonucleotide
<400> 77
   acgccgacgt cgtctgtggt ggggtgtcta ccgccaacgc gacgg 45
<210> 78
   <211> 45
   <212> DNA
   <213> Synthetic Oligonucleotide
<400> 78
   cgactacaac ggctgggata tcaacacccc ggcgttcgag tggta 45
<210> 79
   <211> 6
   <212> DNA
   <213> Synthetic Oligonucleotide
<400> 79
   ccaccc 6
<210> 80
   <211> 5
   <212> DNA
   <213> Synthetic Oligonucleotide
<400> 80
   cggta 5
<210> 81
   <211> 11
   <212> DNA
   <213> Synthetic Oligonucleotide
<400> 81
   gtgtgtttgg t 11
<210> 82
   <211> 11
   <212> DNA
   <213> Synthetic Oligonucleotide
<400> 82
   ggttttgttt g 11
<210> 83
   <211> 12
   <212> DNA
   <213> Synthetic Oligonucleotide
<400> 83
   ttgttttttt tg 12
<210> 84
   <211> 4
   <212> PRT
   <213> Synthetic Peptide
<400> 84
   Tyr Val Ala Asp
   1
<210> 85
   <211> 4
   <212> PRT
   <213> Synthetic Peptide
<400> 85
   Asp Glu Val Asp
   1
<210> 86
   <211> 5
   <212> PRT
   <213> Synthetic Peptide
<220>
   <221> SITE
   <222> (4)..(4)
   <223> X = Any Amino Acid
<400> 86
   Ile Leu Glu Xaa Cys
   1 5
<210> 87
   <211> 4
   <212> PRT
   <213> Synthetic Peptide
<400> 87
   Ile Glu Gly Asp
   1